(19) Europäisches Patentamt / European Patent Office / Office européen des brevets

(11) **EP 4 362 988 B1**

(12) **EUROPEAN PATENT SPECIFICATION**


(45) Date of publication and mention of the grant of the patent:
**25.06.2025 Bulletin 2025/26**

(21) Application number: **22737960.9**

(22) Date of filing: **28.06.2022**

(51) International Patent Classification (IPC):
***A61K 49/00*** *(2006.01)* ***C07K 7/06*** *(2006.01)*
***A61P 31/04*** *(2006.01)* ***A61P 31/10*** *(2006.01)*
***A61P 33/04*** *(2006.01)* ***C07K 7/52*** *(2006.01)*

(52) Cooperative Patent Classification (CPC):
**A61K 49/0043; A61K 49/0032; A61K 49/0056; A61P 31/04; A61P 31/10; A61P 33/04; C07K 7/06; C07K 7/52**

(86) International application number:
**PCT/GB2022/051662**

(87) International publication number:
**WO 2023/275537 (05.01.2023 Gazette 2023/01)**


(54) **ANTIMICROBIAL PEPTIDES AND THEIR USE**

ANTIMIKROBIELLE PEPTIDE UND IHRE VERWENDUNG

PEPTIDES ANTIMICROBIENS ET LEUR UTILISATION


(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.06.2021 GB 202109478**

(43) Date of publication of application:
**08.05.2024 Bulletin 2024/19**



(73) Proprietor: **The University of Manchester**
**Manchester, Greater Manchester M13 9PL (GB)**

(72) Inventors:
- **ZHAO, Can**
  **Manchester, Greater Manchester M13 9PL (GB)**
- **BROMLEY, Mike**
  **Manchester, Greater Manchester M13 9PL (GB)**
- **READ, Nick**
  **deceased (ZZ)**
- **VENDRELL ESCOBAR, Marc**
  **South Bridge Edinburgh**
  **Lothian EH8 9YL (GB)**

(74) Representative: **Petty, Catrin Helen et al**
**Venner Shipley LLP**
**St James's**
**79 Oxford Street**
**Manchester M1 6EG (GB)**



(56) References cited:
**WO-A2-2013/169932 ES-A1- 2 191 516**

- **LEE MIN HEE ET AL: "A novel, tomographic imaging probe for rapid diagnosis of fungal keratitis", MEDICAL MYCOLOGY, vol. 56, no. 7, 8 December 2017 (2017-12-08), Abingdon, pages 796 - 802, XP055966526, ISSN: 1369-3786, DOI: 10.1093/mmy/myx125**
- **MUÑOZ ALBERTO ET AL: "Concentration-dependent mechanisms of cell penetration and killing by the de novo designed antifungal hexapeptide PAF26 : Uptake mechanisms of the antifungal peptide PAF26", MOLECULAR MICROBIOLOGY, vol. 85, no. 1, 31 May 2012 (2012-05-31), GB, pages 89 - 106, XP055966515, ISSN: 0950-382X, DOI: 10.1111/j.1365-2958.2012.08091.x**



Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- MENDIVE-TAPIA LORENA ET AL: "Preparation of a Trp-BODIPY fluorogenic amino acid to label peptides for enhanced live-cell fluorescence imaging", NATURE PROTOCOLS, NATURE PUBLISHING GROUP, GB, vol. 12, no. 8, 13 July 2017 (2017-07-13), pages 1588 - 1619, XP037551074, ISSN: 1754-2189, [retrieved on 20170713], DOI: 10.1038/NPROT.2017.048

## Description

### *Field of the Invention*

The present invention relates to antifungal therapeutic and fungal diagnostic compounds in relation to fungal keratitis.

### *Background*

The cornea is the clear, dome-shaped window at the front of the eye and helps to focus light into the eye. Fungal keratitis (FK) is an infection of the cornea caused by fungi. There is an increasing incidence of FK globally - around 1.5 million people every year are diagnosed with FK, especially in tropical climates such as in South East Asia (Brown et al., 2021). FK can develop after an eye injury or contact lens use. It can cause blindness if it is not treated promptly. In many cases, treatment cannot restore vision, and permanent vision impairment or blindness may occur. Around 50% of FK patients with moderate to severe FK lose sight, either by becoming blind or severely visually impaired. Around 25% of patients with corneal infections require removal of the eye.

Early diagnosis and treatment are critical to minimize tissue damage and ensure the best possible vision outcome (Bacon et al., 1993). Differentiation of fungal from other causes of microbial keratitis is difficult as the clinical features are non-specific. A patient will typically be treated with antibacterial agents initially and will receive an antifungal agent if initial antibacterial treatment is ineffective or if a definitive diagnosis of fungi is made, usually based upon microbiological testing.

There are currently three methods used to make a definitive diagnosis of FK: clinical examination, microbiological testing of corneal scrapings and *in vivo* confocal microscopy (IVCM). However, each of these methods has limitations. Clinical examination relies upon presence of characteristic features for FK (e.g. satellite lesions) that usually are not present in early ulcers, and even if they are present these features can be difficult to distinguish with experienced ophthalmologists recognising these signs only 2/3 of the time (Dalmon et al., 2012). Corneal scraping that is performed for microbiological testing, is an invasive procedure, and only obtains organisms present in the superficial ulcer and in some settings is only able to detect an organism in 40% of cases (Burton et al., 2011). Superficial corneal scraping may miss organisms that are only present in deeper tissue, such as in moderate to severe FK where infection involves the posterior half of the cornea. Fungal culture results can take up to 14 days, and this time delay before the correct treatment regimen can be started can contribute to significant corneal tissue damage and ultimately poor visual outcome for the patient. Also, many regions of the world do not have access to a microbiological lab with the facilities required for fungal culture. The final diagnostic technique, IVCM, is non-invasive and allows for rapid diagnosis at the first clinic visit. However the equipment is expensive and so is not readily available throughout the world. IVCM also requires extensive training in image acquisition and interpretation in order to be able to make a diagnosis of FK (Chidambaram et al., 2016). As such, it is being used mainly in research centres globally rather than in daily routine practice.

Recent advances in molecular biology techniques have allowed for culture-independent diagnostic methods. One such technique is PCR, which has been shown to be useful for the culture-independent diagnosis of various microbial infections, including mycoses. However, a successful amplification of fungal DNA from the corneal scrapings by PCR still requires at least a 48 h post-inoculation period, and presence of a lab with the specialist equipment. This technique is used in the research setting at present, and has not become a test that is routinely used in clinical practice as yet.

The current clinical pathways to diagnose fungal eye infections in developed countries and LMICs are as follows with UK and India used as examples:

- UK (Tuft et al., 2013): clinical features of FK if present are identified at slit lamp examination by the ophthalmologist, normally followed by taking a corneal scraping. Microbiological culture takes approximately 7-14 days for fungi and if positive patients are normally treated with Natamycin 5% eyedrops (which are produced in the US, are expensive and have a time delay for import). Empirical treatment is initiated, whilst awaiting microbiology test results, and may include antibiotic eyedrops +/- antifungal eyedrops depending on whether any clinical features of FK were detected. Voriconazole 1% eyedrops (most frequently available in preservative-free formulation) can also be used to treat fungal eye infections but this has to be ordered as needed due to stability issues, so may take a few days to become available to the patient. *In vivo* confocal microscopy can also be used if available to give an immediate diagnosis, however these machines are expensive and there are only approximately 10 in the UK.

- India: Patients are more likely to visit the ophthalmologist at a later stage of FK and so it is often more difficult to diagnose based on clinical features (Chidambaram et al., 2018). As in the UK, the patient is likely to initially receive an antibiotic eyedrop as well as an antifungal eyedrop whilst awaiting microbiological results. Very few eye clinics in India have an *in vivo* confocal microscope (approximately 6), however if available, this can be used at the first clinic visit to

make a rapid diagnosis of fungi within the ulcer. Natamycin 5% and Voriconazole 1% eyedrops are manufactured locally in India and are readily obtainable at a low-cost.

Perlin et al. developed a modified Caspofungin molecule that has a fluorescent probe that is visualized using the FMT 1500 *in vivo* imager system (near infrared 680 nm wavelength laser) (Perlin et al., 2018). Caspofungin is used as a fungal treatment for systemic fungal disease, but is not as yet available in eyedrop formulation worldwide (Neoh et al., 2014). Caspofungin has some activity against *Aspergillus* species, with moderate MICs noted against clinical isolates. However, Caspofungin is not particularly active against *Fusarium* species (as shown in MIC testing in clinical isolates) (Arikan et al., 2001). Perlin is also an inventor for EP 2846840 A2, which describes methods to detect a fungal cell using antifungal drugs, including Caspofungin, Anidulafungin and Micafungin, bound to a detectable label, such as a fluorescent label.

Lee et al. developed a modified Moxifloxacin molecule to label fungal cells (Lee et al., 2018). Moxifloxacin is a broad spectrum antibiotic. The modified Moxifloxacin molecule lacks specificity as it appears to bind to other corneal cells such as dendritic cells, corneal nerves and possibly keratocytes, and may target bacteria.

There is a need to develop a highly specific and sensitive diagnostic tool enabling a rapid and reliable detection of fungal keratitis, without using any special or costly medical equipment, so this technology can be easily introduced and adopted globally.

There is also a need to develop novel antifungal therapeutics as available drugs have poor solubility, and their efficacy is limited by lack of penetration into the corneal tissue. For FK treatment, Natamycin 5% eyedrops are considered to be the first line treatment for filamentous fungi such as *Fusarium* sp. and *Aspergillus* sp. (Oldenburg et al., 2017). Voriconazole 1% eyedrops are also used but are less effective than Natamycin (Prajna et al., 2013) for filamentous fungi. Both drugs have poor outcomes in treatment of *Aspergillus Keratitis* and deep fungal ulcers, and so there remains an unmet need for novel antifungal eye drop treatments that are safe and effective therapies for FK.

WO 2013/169932 A2 relates to targeting agents and methods: of 'using the targeting agents to detect a fungal cell in a subject.

Lee at al. (Med Mycol. 2018 Oct; 56(7): 796-802) describes a novel, tomographic imaging probe for rapid diagnosis of fungal keratitis.

ES 2191516 A1 describes an anti-fungal antibiotic which which inhibit the germination and growth of the mycellum of phytopathogenic fungi.

Muñoz et al. (Mol Microbiol. 2012 Jul;85(1):89-106. doi: 10.1111/j.1365-2958.2012.08091.x. Epub 2012 May 31) describes the concentration-dependent mechanisms of cell penetration and killing by the de novo designed antifungal hexapeptide PAF26.Mendive-Tapia et al. (Nature Protocols volume 12, pages1588-1619 (2017)) describes the preparation of a Trp-BODIPY fluorogenic amino acid to label peptides for enhanced live-cell fluorescence imaging.

## *Summary of the Invention*

The invention provides compounds and salts thereof and methods of using said compounds and salts in the imaging, diagnosis, and treatment of microbial infections, in particular eye infections.

The compounds of the present invention are of the following general structures:

Cyclo(Arg-Lys-Lys-Xaa-Trp-Phe-Trp-Yaa) **Formula I**

$R^1$-Arg-Lys-Lys-Xaa-Trp-Phe-Trp-Yaa-$R^2$ **Formula II**

It will be understood that the invention encompasses salts of the compounds. Suitable salts of peptides are known in the art.

$R^1$ and $R^2$ may be H and OH, or may be suitable groups as will be apparent to the skilled person. Xaa is an amino acid residue having a hydrophobic side chain. Yaa is an amino acid residue or pair of amino acid residues.

The compounds of the invention may be labelled with a fluorophore, in which case Yaa may comprise a lysine reside bearing a fluorophore in some cases, the fluorophore is attached at the C-terminus, in which case $R^2$ is or comprises a fluorophore. The compounds may be unlabelled, in which case they do not include a lysine reside bearing a fluorophore, although Yaa may be or include a lysine residue.

In a first aspect the invention may provide a compound of Formula I or Formula II or a pharmaceutically acceptable salt thereof, wherein

$R^1$ is H, $C_{1-4}$ alkyl, $C_{1-4}$ acyl, phosphoryl ($PO_3^{2-}$), or biotinyl;

$R^2$ is OH, $NH_2$, H or O($C_{1-4}$ alkyl);

Xaa is a natural or unnatural amino acid residue having a hydrophobic side chain, for example selected from Phe, phe, Ala, ala, Pro, pro, Gly, Val, val, Leu, leu, Ile, ile, Met, met, Tyr, tyr, Trp, and trp;

Yaa is selected from Gly, Lys, Lys-Gly, Lys*, Lys*-Gly, Zaa, Zaa*, Zaa-Gly, and Zaa*-Gly, where Zaa is an unnatural amino acid having a free amine, azide or alkyne side chain, and Zaa* is said unnatural amino acid functionalised with or bearing a fluorophore;

wherein Lys*, if present, is a lysine residue bearing a fluorophore.

It will be understood that when $R^1$ is H the N-terminus of the linear peptides is unsubstituted ($NH_2$ or the protonated form thereof). However, the invention encompasses substitution at this position with, for example, short alkyl groups such as methyl, acyl groups such as acetyl, phosphoryl ($PO_3^{2-}$), or biotinyl groups. The biotinyl group is:

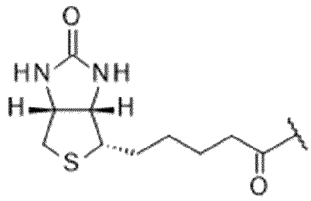

Preferably, $R^1$ is H, $C_{1-4}$ alkyl or $C_{1-4}$ acyl, for example H, methyl (Me) or acetyl (Ac). Most preferably, $R^1$ is H.
When $R^2$ is OH, the C-terminus is the free carboxylic acid, or the deprotonated form thereof. $R^2$ may also be $NH_2$; that is, the linear peptide may terminate in an amide. $R^2$ may also be H; that is, the linear peptide may terminate in an aldehyde. $R^2$ may also be O($C_{1-4}$ alkyl); that is, the linear peptide may terminate in an alkyl ester. Preferably $R^1$ is OH.
In some embodiments, Xaa is selected from Phe, phe, Ala, ala, Pro, pro, Gly, Val, val, Leu, leu, Ile, ile, Met, met, Tyr, tyr, Trp, and trp. In some embodiments, Xaa is selected from phe, ala, pro and Pro. In some embodiments, Xaa is phe. In some embodiments, Xaa is ala. In some embodiments, Xaa is pro. In some embodiments, Xaa is Pro.
In some embodiments, Yaa is selected from Gly, Lys, Lys-Gly, Lys*, and Lys*-Gly. In some embodiments, Yaa is Gly. In these embodiments, the compound or salt is unlabelled. In some embodiments, Yaa is Lys* or Lys*-Gly. In these embodiments, the compound or salt is labelled.
In some embodiments, the compound or salt is a cyclic peptide; that is, according to Formula I. In some embodiments, the compound or salt is a linear peptide; that is, according to Formula II.
In some embodiments the compound is a labelled compound and the fluorophore is selected from Carboxyfluorescein acid and Cyanine5.
In some cases, the compound is selected from the following compounds:

**Table 1**

| Compound | Structure |
|---|---|
| **MFIGAF000** | 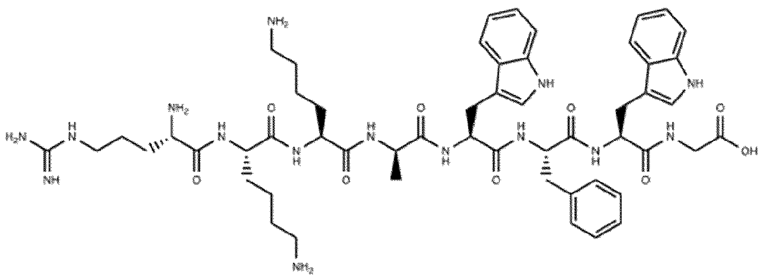<br>$NH_2$-Arg-Lys-Lys-ala-Trp-Phe-Trp-Gly-COOH |

(continued)

| Compound | Structure |
| --- | --- |
| **MFIGAF001** | 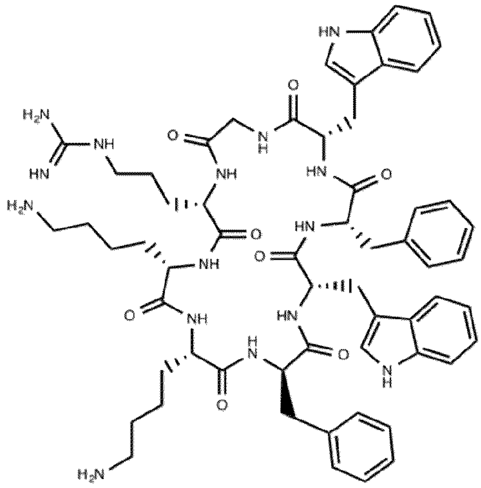<br>Cyclo(Arg-Lys-Lys-phe-Trp-Phe-Trp-Gly) |
| **MFIGAF002** | 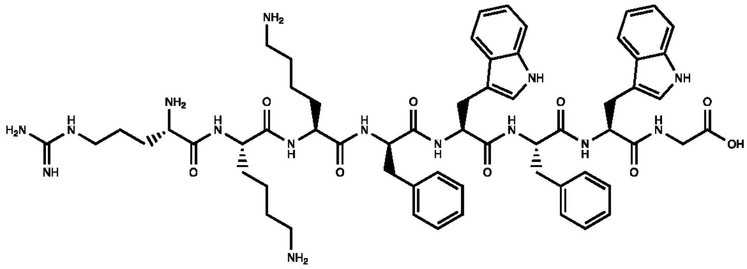<br>$NH_2$-Arg-Lys-Lys-phe-Trp-Phe-Trp-Gly-COOH |
| **MFIGAF003** | 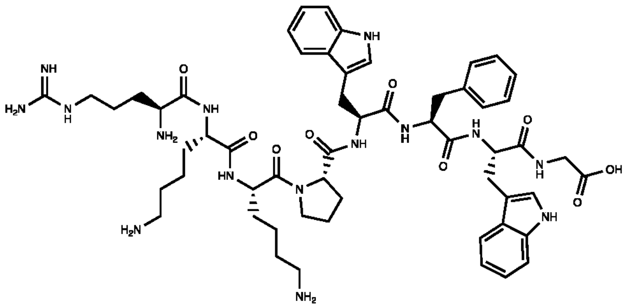<br>$NH_2$-Arg-Lys-Lys-pro-Trp-Phe-Trp-Gly-COOH |
| **MFIGAF004** | 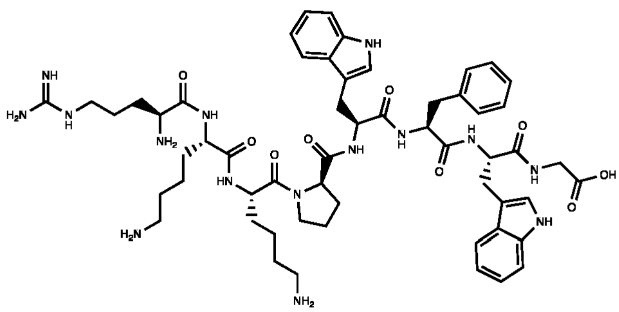<br>$NH_2$-Arg-Lys-Lys-Pro-Trp-Phe-Trp-Gly-COOH |

(continued)

| Compound | Structure |
|---|---|
| **MFIGAF005** | 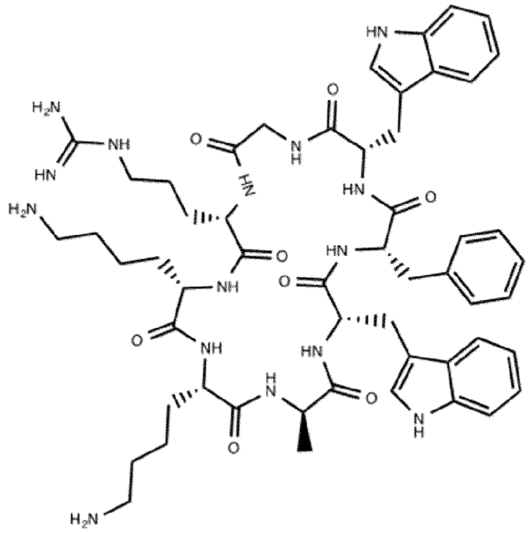 Cyclo(Arg-Lys-Lys-ala-Trp-Phe-Trp-Gly) |
| **MFIGAF007** | 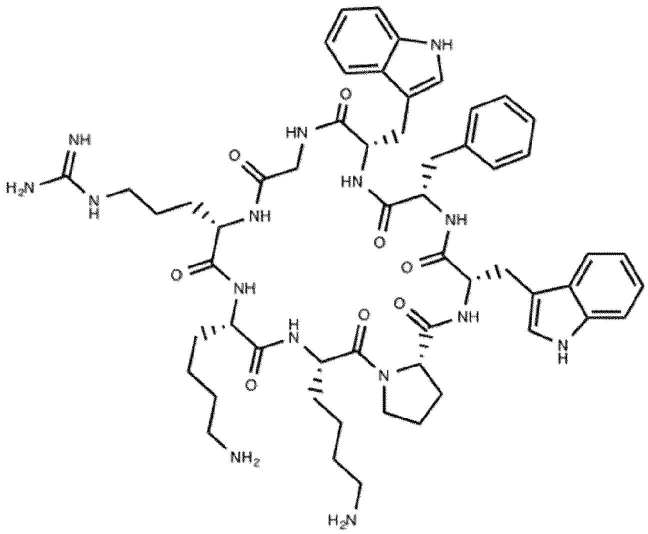 Cyclo(Arg-Lys-Lys-pro-Trp-Phe-Trp-Gly) |

(continued)

| Compound | Structure |
|---|---|
| **MFIGAF008** | 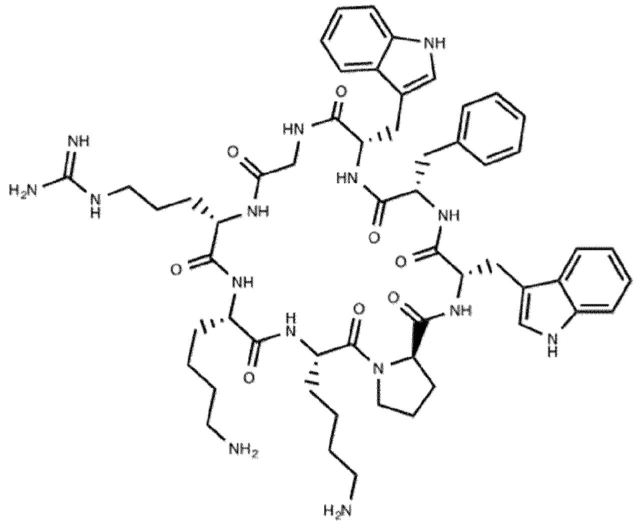 Cyclo(Arg-Lys-Lys-Pro-Trp-Phe-Trp-Gly) |
| **MFIGAF001a** | 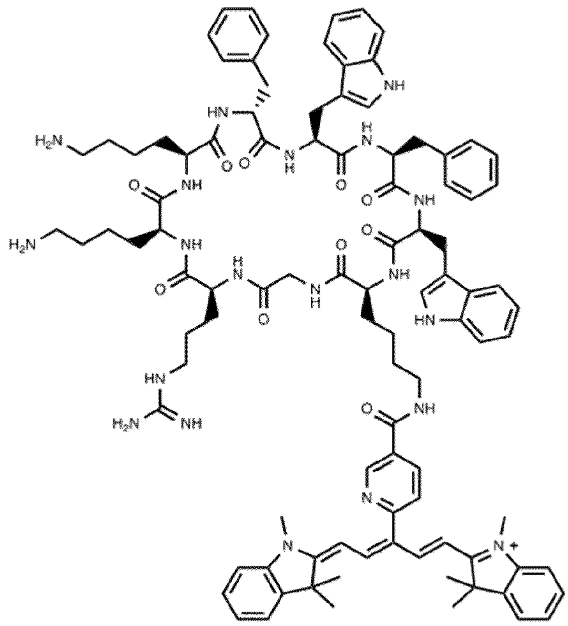 Cyclo(Arg-Lys-Lys-phe-Trp-Phe-Trp-Lys(Cy5)-Gly) |

(continued)

| Compound | Structure |
|---|---|
| **MFIGAF001b** | 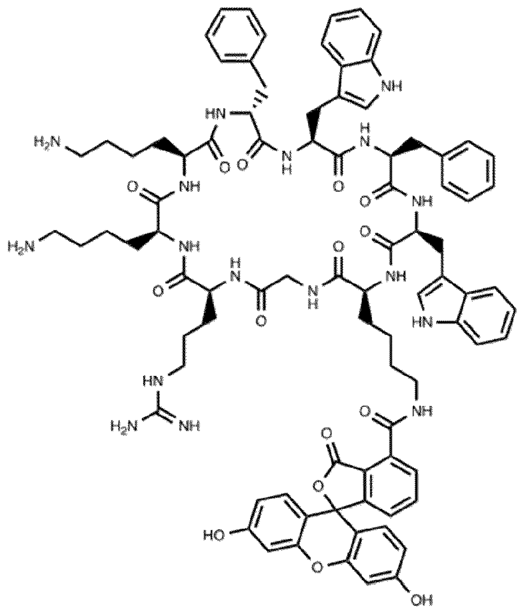 Cyclo(Arg-Lys-Lys-phe-Trp-Phe-Trp-Lys(CF)-Gly) |
| **MFIGAF001c** | 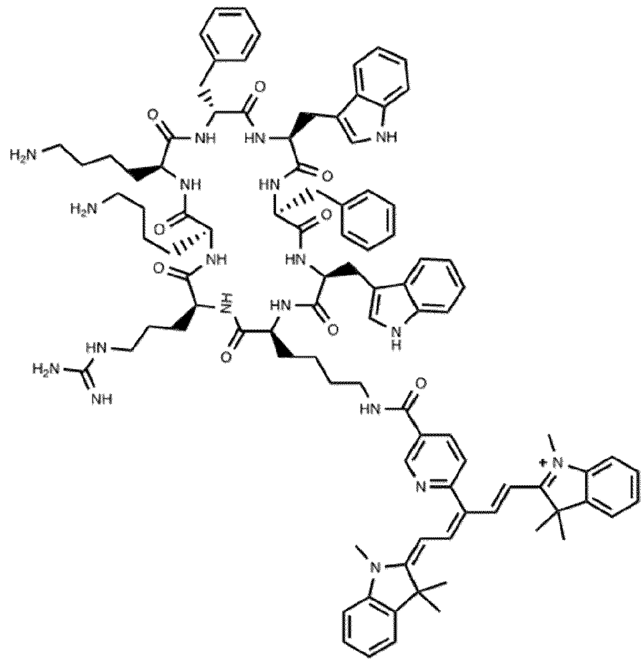 Cyclo(Arg-Lys-Lys-phe-Trp-Phe-Tro-Lys(Cy5)) |

(continued)

| Compound | Structure |
|---|---|
| MFIGAF001d | 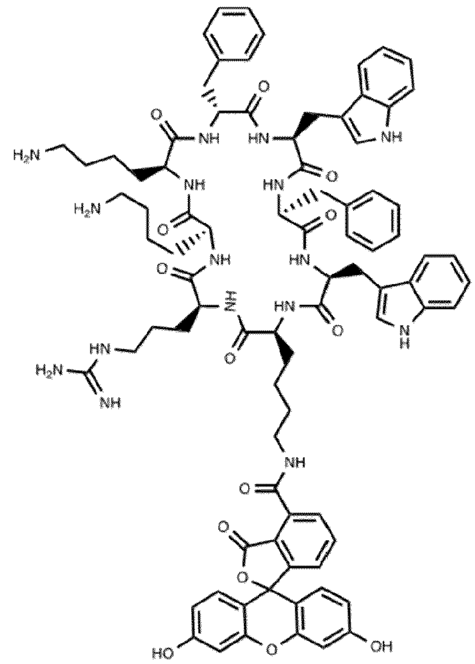 Cyclo(Arg-Lys-Lys-phe-Trp-Phe-Trp-Lys(CF)) |

or a pharmaceutically acceptable salt thereof.

In some embodiments the compound or salt is fluorophore-labelled MFIGAF001.

In a further aspect, the invention provides a pharmaceutical composition comprising a compound or salt of Formula I or Formula II and a pharmaceutically acceptable excipient. Suitably, the composition is formulated for ocular use, for example topical administration to the eye.

In a further aspect, the invention provides a compound or salt of Formula I or Formula II or a pharmaceutical composition comprising such a compound or salt for use in a method of treatment.

The method of treatment is suitably of a microbial infection, for example a fungal infection, a bacterial infection, or an amoebic infection. Suitably, the infected tissue is the eye. The infection may be selected from fungal keratitis (FK), bacterial keratitis or acanthamoeba keratitis. Preferably, the infection is FK.

The inventors have shown that both unlabelled and unlabelled compounds have desirable activity against these infections. Accordingly, both labelled and unlabelled compounds may be used in treatment. However, it will be understood that unlabelled compounds are likely to be preferred for treatment courses.

The labelled compounds of the invention may be used for imaging and diagnosing such infections, and advantageously successful imaging indicates that the labelled and corresponding unlabelled compound is useful in the treatment of the infection, reducing the unnecessary use of ineffective antimicrobial agents which may give rise to the development of antimicrobial resistance in communities.

In a further aspect therefore, the invention relates to methods of diagnosis of microbial infections and may provide a compound or salt of Formula I or Formula II wherein Yaa is Lys*, Lys*-Gly, Zaa*, or Zaa*-Gly, or a pharmaceutical composition comprising such a compound or salt, for use in a method of diagnosing a fungal, bacterial or amoebic infection in the eye of a patient, the method comprising:

a) contacting the compound, salt or composition with the patient's eye;
b) inspecting the patient's eye to determine if fluorescence is detectable; and
c) noting the fluorescence, if present, and diagnosing the patient with a fungal, bacterial or amoebic infection.

The method may further comprise treating the infection with a compound of the invention which may the same or different to the compound used for diagnosis. The type of infection may be determined by examining the morphology of the fluorescent areas.

In a further aspect, the invention provides a method of imaging microbial growth in tissue using a compound or salt of of Formula I or Formula II wherein Yaa is Lys*, Lys*-Gly, Zaa*, or Zaa*-Gly, or a pharmaceutical composition

comprising such a compound or salt, the method comprising:

a) contacting the compound or composition with the tissue;
b) detecting fluorescence of the compound or composition.

The type of microbial growth may be determined by examining the morphology of the fluorescent areas.
It will be appreciated that methods may be *in vivo,* in which case the compound or salt will typically be provided as a pharmaceutical composition such as an eye drop formulation, *ex vivo* or *in vitro.*

*Contribution to the art*

Recognising the difficulties associated with effective diagnosis and treatment of FK, the inventors set out to provide a rapid, non-toxic, broad spectrum and fungal specific fluorescent diagnostic probe rapidly to diagnose fungal keratitis.
Compared to currently available diagnostic approaches, the inventors believe that labelled compounds described herein have the potential to be cost effective and only require minimum training and equipment. It is envisaged that the diagnostic probes will allow direct visualisation of fungi in the human cornea, facilitating rapid diagnosis in the clinic. Since the molecules use the same fluorescent wavelengths for excitation/emission that are employed by other widely used eyedrop diagnostics, visualisation of the probe can be performed through use of a slit lamp biomicroscope, which is used as the standard equipment in routine practice by eyecare professionals such as optometrists, opticians, ophthalmic nurses and ophthalmologists.
It is envisaged that labelled compounds may be provided in a diagnostic kit, that includes an eye drop, and may also include a handheld light source. The anticipated usage of the kit will be to provide a rapid and accurate diagnosis of fungal keratitis; only minimal training will be required to use this kit. In use it is expected that the patient will have the probe applied in the same way as an ordinary eye drop. Any potential fungal infection would be visualised in format of fluorescent signals after a short period (for example, 5 to 20 min), using the slit lamp biomicroscope or suitable handheld light as the excitation light source and method for detection.
The compounds described herein (both labelled and unlabelled) also show desirable biological activity and may be used in methods of treatment as described herein.
The invention includes the combination of the aspects and preferred features described except where such a combination is clearly impermissible or expressly avoided.

***Summary of the Figures***

Embodiments and experiments illustrating the principles of the invention will now be discussed with reference to the accompanying figures in which:

**Figure 1** shows contains HPLC graphs showing the purity of MFIGAF001 and analogue PAF26 after treatment with *Streptomyces griseus* protease at 37°C. Figure 1a) is a graph of the purity of MFIGAF001 at 0 h. Figure 1b) is a graph of the purity of MFIGAF001 at 24 h. Figure 1c) is a graph of the purity of analogue PAF26 at 0 h. Figure 1d) is a graph of the purity of analogue PAF26 at 24 h.

**Figure 2** shows is a graph of the viability of human lung A549 epithelial cells after 4h incubation with different concentrations of MFIGAF001. PBS was used as the negative control and DMSO was used as the positive control. Data is represented as means ±s.d. with n=3. No significant differences ($p > 0.05$) were determined between the negative control and any of the treatments.

**Figure 3** shows is a graph of the haemolytic activity of 10 μM MFIGAF002, MFIGAF001a and MFIGAF001 in human red blood cells (RBCs). Molecules, DMSO (as positive control) and PBS (as negative control) were incubated with human RBCs at 37°C and their viability was assessed after 1 h.

**Figure 4** shows viability reconstructed cornea-like epithelium after incubation with compounds described herein. Figure 4a is a graph of the viability reconstructed cornea-like epithelium (RhCE, EpiOcular™) after incubation with MFIGA001. NC = Negative Control; PC = Positive Control. Figure 4b is a graph of the viability reconstructed cornea-like epithelium (RhCE, EpiOcular™) after incubation with 10 μM of MFIGA001a and MFIGAF001b. NC = Negative Control; PC = Positive Control; A2 = MFIGAF001a; and B1 = MFIGAF001b.

**Figure 5** shows a graph of the tolerability of MFIGAF001a in rabbit eye. Three rabbits (rabbits 1, 2 and 3) were tested.

All rabbits appeared unaffected immediately post-treatment and through the next 7 days with no signs of irritation and no change in general condition. MFIGAF001a was used at 10 μM and 50 μL and was admitted into the right eye of the rabbits.

**Figure** 6 contains images of ex *vivo* corneas infected with YFP-*A*. *fumigatus* treated with either PBS or MFIGAF001. All cornea were treated with one drop of reagent every 2 hours for the first 8 hours, then one drop every 8 hours for the remaining 16 hours. Treatment was continued for 72 hours with this treatment regimen. MFIGAF001 was used at 100 mg/L.

**Figure 7** contains images of an *ex vivo* cornea infected with A. *fumigatus* treated with MFIGAF001, using the fluorescent confocal live-cell imaging approach with a yellow fluorescent protein (YFP). The yellow fluorescent signal (yellow in the original graphs, white reproduced here) indicates the live fungal burden that penetrated into the corneal tissue. All cornea were treated with one drop of reagent every 2 hours for the first 8 hours, then one drop every 8 hours for the remaining 16 hours. Treatment was continued for 72 hours with this treatment regimen. MFIGAF001 was used at 100 mg/L.

**Figure 8** contains images of an *ex vivo* cornea infected with A. *fumigatus* treated with either Natamycin or MFIGAF001, using the fluorescent confocal live-cell imaging approach with a YFP. All cornea were treated with one drop of reagent every 2 hours for the first 8 hours, then one drop every 8 hours for the remaining 16 hours. Treatment was continued for 72 hours with this treatment regimen. The yellow fluorescent signal (yellow in the original graphs, white reproduced here) indicates the live fungal burden that penetrated into the corneal tissue. MFIGAF001 was used at 100 mg/L. Natamycin was used at 120 mg/L.

**Figure 9** shows live cell imaging of MFIGAF001a and MFIGAF001b (fluorescent, 2.5 μM) after 15 min incubation at 37°C in RPMI with various pathogenic fungi (C. *albicans, F.oxysporum, A. fumigatus,* and *C*. *neoformans*). The top panel shows the fluorescent images of fungal cells labelled with MFIGAF001a/b, and the bottom panel shows the brightfield images of fungal cells.

**Figure 10** shows live cell imaging of MFIGAF001a (fluorescent, 2.5 μM) incubated at 37°C in RPMI with *A. fumigatus.*

**Figure 11** shows live cell imaging of A. *fumigatus* in co-cultures with human epithelial cell line A549, 10 min after addition of MFIGAF001a (fluorescent, 2.5 μM) at 37°C in RPMI. The top image shows the brightfield image of A. *fumigatus* hyphae (left) together with A549 cell (right), the bottom image shows the fluorescent chanel of the same view and demonstrates MFIGAF001a selectively labels A. *fumigatus* over A549 cell.

**Figure 12** shows live cell imaging of MFIGAF001a (fluorescent, 2.5 μM, 15 min incubation at 37°C) selectively labelled A. *fumigatus* in a pig corneal infection model. (A) fluorescent image shows MFIGAF001a selectively labelled A. *fumigatus* (fluorescent filaments) over porcine corneal tissue (dark background); (B) shows the porcine corneas infected with A. *fumigatus;* (C) brightfield image shows A. *fumigatus* growing within porcine corneal tissue.

**Figure 13** shows live cell imaging of MFIGAF001a (fluoresent, 2.5 μM) incubated at 37°C in RPMI for 1 h with P. *aeruginosa.* The top image shows the fluorescent channel indicating P. *aeruginosa* not being labelled by MFIGAF001a, and the bottom image shows the brightfield image of P. *aeruginosa.*

**Figure 14** shows live cell imaging of MFIGAF001a (fluoresent, 2.5 μM, 20 min incubation at 37°C) in RPMI with S. *aureus.* The top image shows the fluorescent channel indicating S. *aureus* being labelled by MFIGAF001a, and the bottom image shows the brightfield image of S. *aureus.*

**Figure 15** shows live cell imaging of MFIGAF001a (fluorescent, 2.5 μM, 15 min incubation at 37°C) in a pig corneal infection model co-infected by A. *fumigatus* and S. *aureus.* The top image shows the fluorescent channel shows the size differences of A. *fumigatus* (filamentous structures) and S. *aureus* (dots) being labelled by MFIGAF001a over the corneal tissue, and the bottom image shows the brightfield image of the co-infected cornea.

### *Detailed Description of the Invention*

Aspects and embodiments of the present invention will now be discussed with reference to the accompanying figures. Further aspects and embodiments will be apparent to those skilled in the art.

*Amino acid nomenclature*

Amino acids and amino acid residues (amino acid moieties within a peptide chain) are referred to by their conventional three letter or single letter codes. Capitalisation indicates the naturally occurring L-amino acids, while lower case denotes the corresponding D-amino acids. For example, Pro and P denote L-proline, while pro and p denote D-proline.

Linear peptides are written from N-terminus to C-terminus, left to right.

Cyclic peptides are indicated by the term "cyclo" in front of the sequence.

Unnatural amino acids and unnatural amino acid residues are amino acids and amino acid residues that do not naturally occur in peptide chains. Unnatural amino acids may be formed as secondary metabolites in bacteria, fungi, plants, or marine organisms, or they can be synthesised chemically.

Residues marked with an asterisk are labelled as described herein. That is, Lys* denotes a labelled lysine amino acid or residue. As described herein, Lys* is labelled with fluorophore. The fluorophore is attached at the primary amine of the lysine moiety, for example by an amide bond. Where the fluorophore is specified an abbreviation is provided in parentheses immediately after the residue and the asterisk is omitted. For example, Lys(CF) and K(CF) denote lysine labelled with carboxyfluorescein acid.

*Labelled Compounds and methods of diagnosis*

Two types of related compound are described herein, referred to as labelled compounds and unlabelled compounds. Labelled compounds bear a fluorophore which may be useful in the diagnosis of eye infections such as fungal keratitis (FK). The labelled compounds may be provided as an eye drop, and when applied bind to the infected area and so, after a short period (for example, up to 20 minutes), an infection can be imaged by detecting fluorescence of the label. This is straightforward to detect using a slit lamp biomicroscope or similar. Most eye clinics, hospital eye departments, and optometry practices worldwide have a slit lamp biomicroscope with fluorescent light capabilities as this is used by eyecare professionals in daily routine practice. Of course, another suitable light source and optionally separate magnifier may be envisaged.

The labelled compounds of the invention and salts thereof, or pharmaceutical compositions containing said compounds or salts may be for use in a method of diagnosing a microbial infection, for example an eye infection.

A method diagnosis of an eye infection in a patient may comprise the following steps:

a) applying the compound, salt, or composition to the patient's eye;
b) inspecting the patient's eye to determine if fluorescence is detectable; and
c) noting the fluorescence, if present, and diagnosing the patient with an eye infection.

Accordingly, compositions comprising labelled compounds suitable for applying to the eye (for example, in the form of an eye drop) may be provided in a diagnostic kit.

The inventors have found that the compounds of the invention bind to fungi such as FK, certain bacteria, and amoeba. The labelled compounds of the invention may therefore be used to detect the presence of any such infection.

In some cases, the diagnosis is of a fungal infection, such as FK. In some cases, the diagnosis is of a bacterial infection, such as gram-positive or gram-negative bacterial infection ,for example bacterial keratitis. In some cases, the diagnosis is of an amoebic infection.

In other words, in some cases the eye infection is a fungal eye infection. In some cases the eye infection is a bacterial eye infection. In some cases the eye infection is associated with amoeba, for example an amoebic infection such as acanthamoeba keratitis.

Accordingly, it will be appreciated that the practioner will appreciate that an absence of fluorescent areas may be used to determine that the patient's eye is uninfected or infected with a pathogen to which the compounds of the invention do not bind. For example, an absence of fluorescence may be used to determine that the patient does not have FK.

Advantageously, the inventors have found that labelled compounds of the invention show desirable biological activity analogus to the unlabelled compounds, and therefore have additional utility in the treatment of infections. As a result, the diagnostic test itself may initiate treatment of the infection immediately. A compound of the invention (or other suitable agent) may then be prescribed if appropriate. It will be appreciated that the prescribed treatment will normally but not necessarily be an unlabelled compound.

By observing and interpreting the morphology associated with the fluorescence, the type of infection may be determined.

Accordingly, in some cases the invention provides a compound of the invention for use in a method of diagnosis of an eye infection in a patient comprising the following steps:

a) applying the compound, salt, or composition to the patient's eye;
b) inspecting the patient's eye to determine if fluorescence is detectable; and
c) noting the fluorescence and, if present, observing the morphology of areas of fluorescence;
d) diagnosing the patient with an infection based on the presence of fluorescence and categorising the infection as fungal, bacterial or amoebic based on the morphology.

The inspection will typically be after a period of time as described herein, and will use a slit lamp biomicroscope or similar device (or a suitable light source and optionally separate magnifier). Observing the morphology may make use of confocal microscopy.

In some preferred cases, the eye infection is a fungal eye infection such as FK. Advantageously, the inventors have found that labelled compounds of the invention show desirable anti-fungal activity, and therefore have additional utility in the treatment of FK. As a result, the diagnostic test itself may initiate treatment of the FK immediately. A compound of the invention (or other antifungal) may then be prescribed if appropriate. It will be appreciated that the prescribed treatment will normally but not necessarily be an unlabelled compound.

The compound of the invention may be used in a method of diagnosis of FK in a patient comprising the following steps:

a) applying the compound, salt, or composition to the patient's eye;
b) inspecting the patient's eye to determine if fluorescence is detectable; and
c) noting the fluorescence, if present, and diagnosing the patient with fungal keratitis.

The compound, salt or composition may be administered to the patient as an eye drop. It may be followed by waiting a period after administration before inspecting the patient's eye, for example, waiting 5 to 30 minutes, preferably 5 to 20 minutes, for example 10 to 20 minutes. Inspecting the patient's eye is done visually using for example a slit lamp, as will be familiar to the skilled person. In other words, step (b) may include illuminating the patient's eye then visually inspecting it. The labelled compound binds to the fungus, if present, and can be observed as shown in the accompanying examples. It will be appreciated that such observation using a trace or dye is familiar to skilled persons such as opticians, optometrists, ophthalmic nurses and ophthalmologists.

Importantly, the inventors have found that the binding and therefore detection is specific for infections for which the compound is active, and is especially useful for the detection of fungal infections, such as FK, ensuring that treatment is appropriate. This may help to avoid unnecessary use of antibiotics, which is an important consideration in managing the prevalence of antimicrobial resistance and the emergence and proliferation of resistant microbial strains.

*Methods of imaging*

The compounds of the invention, salts, or pharmaceutical compositions thereof may be for use in a method of imaging microbial growth in tissue. The compounds, salts or pharmaceutical compositions thereof for use in a method of imaging microbial growth in tissue are wherein Yaa is Lys*, Lys*-Gly, Zaa*, or Zaa*-Gly, such that the compounds, salts or the pharmaceutical compositions are fluorophore-labelled.

The method of imaging microbial growth in tissue comprises:

a) contacting the compound or composition with the tissue;
b) detecting fluorescence of the compound or composition.

In some cases, the growth is of a fungas, such as the fungus associated with FK. In some cases, the growth is bacterial, such as gram-positive or gram-negative bacterial growth. In some cases, the growth is amoebic.

It will be appreciated that the imaging may be used to determine the presence of growth, which may be useful in the diagnosis of an infection as described above in a patient. The imaging may also be in a laboratory as an *in vitro* method, or *ex vivo,* for example for research purposes or during post-mortem examination. The type of growth may be determined by observing and interpreting the morphology of the fluorescence. Observing the morphology may make use of confocal microscopy (e.g. a slip lamp biomicroscope) or similar although other imaging and magnification techniques may be used as appropriate and known in the art.

Accordingly, the present invention may provide use of a labelled compound as described herein in the imaging of microbial growth, for example fungal, bacterial or amoebic growth *in vivo, ex vivo,* or *in vitro.*

*Fluorophores*

The labelled compounds of the invention bear a fluorophore. The term fluorophore is understood in the art and

refers to is a fluorescent chemical compound or moiety that can re-emit light upon light excitation.

As MFIGA001 has the most potent antifungal activity *in vitro,* it was considered the most appropriate candidate to assess as a diagnostic, but it will be appreciated that other compounds described herein may be labelled in the same way.

Suitable fluorophores for use in preparing labelled compounds as described herein include Cyanine5 and carboxyfluorescein. Other cyanine dyes are also envisaged, for example Cy 5.5, or Cy7. Other commercially available fluorophores with emission and excitation wavelengths that meet international standards and safety requirements for ophthalmic usage (such as the ANSI Z80.36-2016 standard for ophthalmic instruments, or the ISO 15004-1 & 15004-2:2007 standards) may also be used for labelling compounds of the invention, for example MFIGAF001.

Means of attachment to the amine group will be recognised by the skilled person, but may include amide bond formation, sulphonamide bond formation, and leaving group displacement to generate a secondary amine.

Preferred fluorophores are Cyanine5 and carboxyfluorescein, which are attached via amide bond.

<u>*Methods of treament*</u>

The compounds (both labelled and unlabelled) and salts thereof, or pharmaceutical compositions containing said compounds or salts may be for use in a method of treatment.

The method of treatment may be a method of treatment for a fungal infection, such as fungal keratitis. The method of treatment may be a method of treatment for a bacterial infection, such as gram-positive or gram-negative bacterial infection. The method of treatment may be a method of treatment for an amoebic infection. The method of treatment may be a method of treatment for a microbial infection.

<u>*Pharmaceutical compositions*</u>

The compounds of the invention or salts thereof may be provided in pharmaceutical compositions. In other words, the invention provides a pharmaceutical composition of the invention comprising a compound or salt of the invention and a pharmaceutically acceptable excipient.

Suitably, the pharmaceutical composition is formulated for administration to the eye, for example as an eye drop. The pharmaceutical composition may therefore be provided in an eye dropper bottle, or in a bottle with a pipette, or in single-use eye drop containers.

The pharmaceutical composition may be used for detection/diagnosis and/or treatment as appropriate and may be labelled accordingly or accompanied by suitable instructions.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

***Synthetic Examples***

*Chemicals*

Unless stated otherwise, all chemicals were purchased from Sigma-Aldrich (https://www.sigmaaldrich.com). All purchased commercially available reagents were used without further purification.

*Vessels*

Unless stated otherwise, all organic chemical reactions were performed in dried clean glassware under nitrogen. All peptide synthesis reactions were performed in polystyrene syringes fitted with a polyethylene porous disc. All microwave reactions were carried out using focused mono-mode microwave oven ('Discover' by CEM Corporation) with a surface sensor for internal temperature determination. Cooling was provided by compressed air ventilating the microwave chamber during the reaction.

*Thin layer chromatography*

For monitoring the progression of reactions or purification progression, thin layer chromatography (TLC) was performed on Merck silica gel 60 F254 sheets and visualized by ultraviolet (UV) light at 254 nm and 365 nm wavelengths.

*HPLC instrumentation*

**Analytical HPLC:**

Analytical high performance liquid chromatography (HPLC) analysis was conducted on an Agilent 1100 separations module connected to a multiwavelength UV detection system. The parameters used for analytical HPLC were:
Stationary phase: Symmetry C18 column, 4.6 × 150 mm, 5 μm particle diameter size. Mobile phase: A: water + 0.1 % formic acid (FA), B: acetonitrile + 0.1% FA. A linear mobile phase gradient was used with a flow rate of 1 mL/min. Standard sample preparation: 10 μL injection of 1 mg/mL concentrated sample solution in MeOH. Detection: absorbance detection from 280 nm up to 650 nm depending on the sample. Retention times ($t_R$) were noted in minutes.

*Preparative HPLC*

Peptide crudes were purified using an Agilent 1100 semi prep HPLC system unless otherwise stated. The parameters used for preparative HPLC were:
Stationary phase: *Agilent semi-prep* C18 column, 21.2×150 mm, 10 μm diameter size. Mobile phase: A: water + 0.1 % FA, B: acetonitrile + 0.1% FA. A linear mobile phase gradient was used with a flow rate of 20 mL/min. Standard sample preparation: Multiple 50 μL injections of 10 mg/mL concentrated sample solution in water. Detection: absorbance detection from 280 nm up to 650 nm depending on the sample.

*Nuclear magnetic resonance (NMR) instrumentation*

$^1$H-NMR and $^{13}$C-NMR spectra were recorded on a *Bruker Avance 500* (500 MHz) or *Avance 400* (400 MHz) instrument. Chemical shifts were reported in δ ppm downfield from the internal standard $Me_4Si$ (δ = 0 ppm); *J* values were provided in Hz. The multiplicities were reported by the following symbols: s (singlet), d (doublet), t (triplet), q (quadruplet), m (multiplet), dt (doublet of triplets), dd (doublet doublets), ps (pseudo singlet), pd (pseudo doublet).

*Mass spectrometry (MS) instrumentation*

Electrospray ionisation (ESI) positive high-resolution mass spectrometry (HRMS) analysis data were obtained with a LTQ-FT Ultra (Thermo Scientific) mass spectrometer. Matrix assisted laser desorption ionisation (MALDI) analysis was performed using a Bruker Ultraflex mass spectrometer.

*SYNTHETIC EXAMPLE 1 - Preparation of fluorescent probes*

*Commercially Available Fluorescent Probes*

Fluorescent probes purchased for this study are listed in Table 2. Dansyl chloride and carboxyfluorescein were purchased from Merck Millipore at 95% purity. The rest of the fluorescent probes were bought from Sigma Aldrich. It will be

understood that these fluorophores may be used as the fluorphore in compounds of the invention.

**Table 2** - Commercially Available Fluorescent Probes

| Abbrev. | Chemical name | Short name | MW | Fluorescence emission colour |
|---|---|---|---|---|
| **DS** | 5-(Dimethylamino)naphthalene-1-sulfonyl chloride | Dansyl chloride | 269 | Blue-Green |
| **CF** | 3',6'-Diacetoxy-3-oxo-3H-spiro[isobenzofuran-1,9'-xanthene]-6-carboxylic acid | Carboxyfluorescein acid | 376 | Green |
| **NBD** | 4-Chloro-7-nitro-1,2,3-benzoxadiazole | Nitrobenzoxadiazole chloride | 182 | Green-yellow |
| **TMR** | N-(4-Carboxy-6'-(diethylamino)-3-oxo-3H-spiro[isobenzofuran-1,9'-xanthen]-3'(9a'H)-ylidene)-N-ethylethanaminium | Carboxytetraethylrhodamine | 485 | Yellow |

***Synthesised Fluorescent Probes***

Fluorescent probes synthesised in this study are listed in Table 3.

**Table 3** - Synthesised Fluorescent Probes

| Abbrev. | Chemical name | Short name | MW | Fluorescence emission colour |
|---|---|---|---|---|
| **DMN** | 8-(Dimethylamino)-3-oxatricyclo[trideca-1,5,7,9,11-pentaene]-2,4-dione | 4-N,N-Dimethylamino-1,8-naphthalimide | 241 | Green |
| **CFA** | 3',6'-Dihydroxy-3-oxo-*N*-(prop-2-yn-1-yl)-3*H*-spiro[isobenzofuran-1,9'-xanthene]-5,6-carboxamide | Carboxyfluorescein alkyne | 413 | Green |
| **MG** | N-(4-((4-carboxyphenyl)(4-(dimethylamino)phenyl) methylene)cyclohexa-2,5-dien-1-ylidene)-N-methylmethanaminium iodide | Malachite Green | 372 | Green |
| **DiAcCFA** | 3-Oxo-6-(prop-2-yn-1-ylcarbamoyl)-3H-spiro[isobenzofuran-1,9'-xanthene] -3',′-diyl diacetate | Di-acetate carboxyfluorescein alkyne | 497 | Green |
| **BODIPY** | 4-(1,3,5,5,7,9-Hexamethyl-5*H*-4$\lambda^4$,5$\lambda^4$-dipyrrolo [1,2-*c*:2',1'-*f*][1,3,2]diazaborinin-10-yl)-N-(prop-2-yn-1-yl)benzamide | Boron-dipyrromethene alkyne | 405 | Green |
| **NB** | N-(5-(5-Carboxypentanamido)-9H-benzo[a]phenoxazin-9-ylidene)-N-ethylethanaminium | Nile Blue | 432 | Yellow-red |
| **SR** | 4-((1E,3E)-4-(4-(dimethylamino)phenyl)buta-1,3-dien-1-yl)-1-(6-ethoxy-6-oxohexyl)pyridin-1-ium bromide | Styryl red | 423 | Orange |
| **SY** | (*E*)-4-(4-(Dibutylamino)styryl)-1-(6-ethoxy-6-oxohexyl) pyridin-1-ium bromide | Styryl yellow | 365 | Orange |
| **Cy5** | 2-((1*E*,3*Z*)-3-(5-carboxypyridin-2-yl)-5-((*E*)-1,3,3-trimethylindolin-2-ylidene) penta-1,3-dien-1-yl)-1,3,3-trimethyl-3*H*-indol-1-ium iodide | Cyanine5 | 521 | Red |

*SYNTHETIC EXAMPLE 1a - Cy5*

**2-((1*E*,3*Z*)-3-(5-carboxypyridin-2-yl)-5-((*E*)-1,3,3-trimethylindolin-2-ylidene)penta-1,3-dien-1-yl)-1,3,3-trimethyl-3*H*-indol-1-ium iodide (Cy5):**

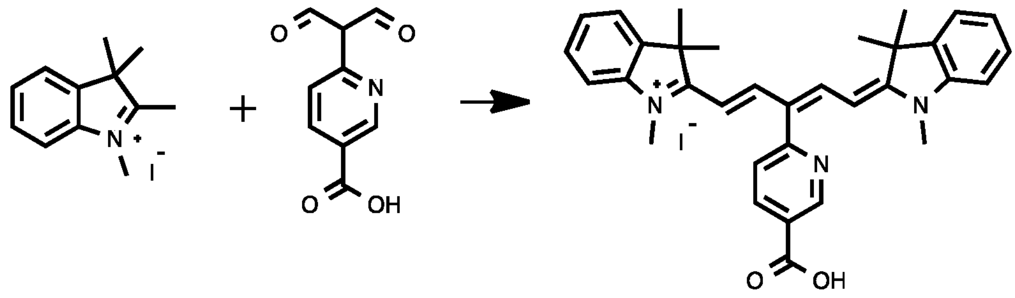

3*H*-Indolium-2,3,3-tetramethyl iodide (198 mg, MW = 274.8, 0.72 mmol), NaOAc (270 mg, MW = 266.1, 0.72 mmol) and 6-(1,3-dioxopropan-2-yl) nicotinic acid (100 mg, MW = 193.2, 0.36 mmol) were added to a microwave reaction vessel with 10 mL $Ac_2O$/AcOH (1:1). The mixture was then heated up to 160 °C under microwave irradiation for 0.5 h, after which the solvent was evaporated under vacuum. The crude was then purified using column chromatography (DCM:MeOH 98:2 to 90:10) to yield 2-((1E,3Z)-3-(5-carboxypyridin-2-yl)- 5-((*E*)-1,3,3-trimethylindolin-2-ylidene)penta-1,3-dien-1-yl)-1,3,3-trimethyl-3*H*-indol-1-ium iodide as a blue solid (144 mg, 80% yield, purity >98%).

**HPLC:** $t_R$: = 4.72 min
**MS** (m/z): [$M^+$] calculated for $C_{33}H_{34}N_3O_2^+$*:* 504.3; found: 504.4.
**$^1$H NMR** (500 MHz, DMSO-$d_6$) δ 9.26 (d, *J* = 1.3 Hz, 1H), 8.44 (s, 1H), 8.41 (s, 1H), 8.40 (d, *J* = 2.2 Hz, 1H), 8.39 (d, *J* = 2.2 Hz, 1H), 7.65 (d, *J* = 7.4 Hz, 1H), 7.56 (d, *J* = 8.0 Hz, 2H), 7.42 (dd, J = 8.0, 1.2 Hz, 2H), 7.40 (d, *J* = 1.1 Hz, 2H), 7.28 (ddd, J = 7.4, 6.5, 1.9 Hz, 4H), 5.85 (d, *J* = 14.3 Hz, 4H), 3.39 (s, 3H), 1.91 (s, 6H), 1.76 (s, 6H).
**$^{13}$C NMR** (126 MHz, DMSO) δ 174.4 (2C), 172.5 (C), 165.0 (C), 151.4 (CH), 143.1 (C), 141.6 (2C), 128.9 (4C), 125.5 (2CH), 122.8 (2CH), 111.8 (2CH), 101.0 (2CH), 95.1 (2CH), 63.3 (2C), 49.6 (C), 31.5 ($2CH_3$), 27.4 ($2CH_3$), 21.6 ($2CH_3$).

*SYNTHETIC EXAMPLE 2 - General preparation of peptides*

**General solid phase peptide synthesis (SPPS) procedure**

Solid phase peptide synthesis consists of assembling amino acids from the C-terminus to the N-terminus. The α-carboxyl group was attached via an acid-labile linker to a solid support. Resins commonly used were composed of polystyrene. The amino-terminus of the amino acid was protected by a base-labile Fmoc (9-fluorenylmethoxycarbonyl) protecting group, whilst the side chains were generally protected with acid-labile groups. After the first amino acid was loaded onto the resin, the Fmoc group was removed under mild basic conditions (Deprotection). A free amine test was then performed to confirm that all of the Fmoc protecting groups were removed. The next Fmoc amino acid was then attached to the growing peptide by activation of its carboxyl group (Coupling). A Kaiser/Bromophenol Blue test was then performed to confirm that complete coupling has occurred on all the free amines on the resin. Synthesis then proceeded through a cycle of deprotection of Fmoc amino terminus groups and coupling of the next amino acid until the peptide had been completely synthesized. The peptide was finally cleaved from the resin and side chain protection groups removed using trifluoroacetic acid (Cleavage).

***Loading of resin***

The majority of resins used in this study were all pre-loaded with a Fmoc protecting group or amino acid. In other cases, e.g. when using 2-chlorotrityl polystyrene resin, the following procedure was performed. Fmoc-amino acid(AA)-OH (1 eq.) was attached to the resin (1 eq.) with DIPEA (3 eq.) in DCM at r.t for 10 min and then DIPEA (7.0 eq.) for 40 min. The remaining trityl groups were capped adding 0.8 μL MeOH/mg resin for 10 min. After that, the resin was filtered and washed with DCM (4 × 1 min), and DMF (4 × 1 min). The loading of the resin was determined by titration of the Fmoc group.

***Determination of resin loading via Fmoc quantification***

Generally, resin loadings were determined after the first synthetic step by measuring the absorbance of the dibenzofulvene-piperidine adduct. Three aliquots of the Fmoc-amino acid resin (~ 1 mg) were weighed precisely and suspended in 1 ml piperidine solution (20% in DMF). After 30 min the mixtures were diluted 1:10 and the absorbance was measured at 290 nm and 330 nm in an *Agilent* 8453 *UV-Vis Spectrophotometer* in the single beam mode with 50 μl UV quartz microcuvettes and using piperidine solution (20% in DMF) as a blank. The resin loading was calculated according to Beer-Lambert Law.

$$L\ (\%) = C_{actual} / C_{theoretical}$$

**while,** $C_{actual} = (A_{290} - A_{blank})/\varepsilon_{Fmoc}$ with $\varepsilon_{Fmoc} = 8100\ M^{-1}cm^{-1}$ at 290 nm **and** $C_{theoretical} = m_{resin} \times L_{resin}$.

L: percentage of loaded resin; $C_{actual}$: concentration of actual Fmoc-piperidine adduct in solution; $C_{theoretical}$: concentration of theoretical Fmoc group in solution; A290: absorbance at 290 nm; m: Amount of resin weighed (mg); $F_{resin}$: resin loading (mmol $g^{-1}$).

***Standard procedure for Fmoc deprotection***

The Fmoc group was removed by shaking the beads in 20% piperidine solution in DMF (1 × 1 min, 2 × 10 min). The beads were then washed extensively with DMF and DCM afterwards. The Bromophenol Blue test and Kaiser test were carried out after each deprotection step. In the case of incomplete deprotection, the procedure was repeated.

***Free amine test***

The Bromophenol Blue test and Kaiser test were carried out after each deprotection step. When the deprotection was found to be incomplete the procedure was repeated.

***Bromophenol Blue Test***

The resin was well washed with DMF before performing this test.

Solution 1: 0.05% bromophenol blue in DMA (store at 25°C)

*Procedure:*

1) Place a few resin beads in a small test tube.
2) Add 5-10 drops of solution.
3) Inspect immediately.

**Table 4** - Bromophenol blue test results indication

| Positive | Partial Positive | Negative |
|---|---|---|
| Blue beads | - | Colourless beads |

***Kaiser Test***

Solution 1: 5 g ninhydrin in 100 mL ethanol (store at 25°C, foiled in the dark)
Solution 2: 80 g phenol in 20 mL ethanol (store at 25°C, foiled in dark)
Solution 3: 2 mL 0.001M KCN in 98 mL pyridine (store at 25°C, foiled in dark)

*Procedure:*

1) Place a few resin beads in a small test tube.
2) Wash resin with ethanol 2-3 times to remove adsorbed DMF.
3) Add 2-5 drops of each solution then mix and heat to 100°C for 2 min.

**Table 5** - Kaiser test results indication

| Positive | Partial Positive | Negative |
|---|---|---|
| Variable intensity blue solution/resin | Blue bead cores | Colourless/light yellow beads |

***Standard procedure for coupling of N-Fmoc amino acids***

After the resin was loaded and the Fmoc group was removed, the resin was washed with DMF (4 × 1 min), DCM (3 × 1 min), and then DMF (4 × 1 min). Unless otherwise noted, a standard coupling procedure was performed using a solution of the appropriate Fmoc-a amino acid (3 eq.), N,N'-diisopropylcarbodiimide (DIC, 6 eq.) and OxymaPure (3 eq.) in DMF solution for 1 h. Five minutes of pre-activation procedure was allowed after mixing the solution before applying on the resin. The completion of the coupling was monitored by the Kaiser test. In the case of positive result, the previous step was repeated. Otherwise, the resin was filtered and washed with DCM (4 × 1 min) and DMF (4 × 1 min) and the following amino acid was coupled in the same way until the peptide reached to its designed length.

***N-terminal acetylation***

After the final coupling step and final Fmoc deprotection, the resin was treated with a solution of $Ac_2O$ (10 eq.) and DIPEA (20 eq.) in DMF. After a reaction time of 1 h at r.t., the resin was drained and again treated with the same amount of fresh reaction mixture for another 1 h. Afterwards, the resin was washed extensively with DMF (4 × 3 min), DCM (4 × 3 min) and dried under vacuum for 12 h.

***Side chain deprotection***

*General side chain deprotection*

Unless otherwise stated, the side chain protecting groups (e.g. tBu, Boc, Trt) were removed during the final cleavage step using an appropriate TFA based cocktail reagent.

***Allyloxycarbonyl (Alloc) deprotection***

Triphenylphosphine ($PPh_3$, 0.8 eq) and palladium acetate ($Pd(AcO)_2$, 0.2 eq.) were mixed with a small amount of DMF and the mixture was sonicated until a vivid light brown coloured, cloudy mixture was formed. Then this mixture together with phenylsilane ($PhSiH_3$, 12 eq.) and 5 mL of dry DCM were added to the resin (2 × 20 min).

***Final cleavage***

*General cleavage for polystyrene (PS) resins*

The resin and the side chain protection groups were removed by stirring the beads in 5 mL appropriate cocktail reagent (TFA based) for 2 h at r.t.. The beads were washed with TFA thoroughly and precipitated with ice-cold diethyl ether after rotary evaporation.

Cleavage cocktail 1: 82.5 : 5 : 5 : 2.5 : 5 - TFA/water/thioanisole/EDT/Phenol
Cleavage cocktail 2: 85 : 5 : 5 : 5 - TFA/water/TIS/Phenol
Cleavage cocktail 3: 95 : 2.5 : 2.5 - TFA/water/TIS
Cleavage cocktail 4: 95 : 5 - TFA/water
*Cleavage of 2-chlorotrityl polystyrene resins*

The resin bound peptide was treated 5 times with 1% TFA in DCM (1 min in each treatment) and washed with DCM. The combined filtered mixtures were poured over DCM and evaporated under vacuum.

*SYNTHETIC EXAMPLE 4 - Preparation of novel rationally designed linear PAF26 derivative peptides*

Linear PAF26 derivatives (named MFIGAF000, MFIGAF002, MFIGAF003, and MFIGAF004) designed in this study were based on computational analysis are listed in Table 7. They were synthesised via SPPS Fmoc chemistry as described before on 2-chlorotrityl polystyrene resins with one extra amino acid inserted in the middle of their sequences.

They were cleaved from the resins using 1% TFA in DCM with all side chains protected. A strong cleavage was then performed using Cleavage cocktail 2 for side chain protecting group removal.

Peptides crudes were purified by semi-preparative RP-HPLC *(Agilent semi-prep* C18 column, 21.2 × 150 mm, 10 μm diameter size) with the mobile phase as ACN (0.1% FA)/$H_2O$ (0.1% FA) and a flow rate of 2 mL·min$^{-1}$. Pure fractions were lyophilised after purification to produce the peptides listed in Table 7.

**Table 6**

| Compound | Structure | Molecular Weight |
|---|---|---|
| **PAF26** | $NH_2$-Arg-Lys-Lys-Trp-Phe-Trp-$CONH_2$ | 949 |

**Table 7** - Characterisation data of nov el design ed linear pep tides

| Compound | Structure | HRMS (m/z) : [M+H]$^+$ | | HPLC $t_R$ (min) | Purity (254 nm) | Amount (mg) |
|---|---|---|---|---|---|---|
| | | Calculated | Found | | | |
| **MFIGAF000** | $NH_2$-RKKaWFWG-COOH | 1154.6 | 1054.7 | 3.68 | 88% | 9.8 |
| **MFIGAF002** | $NH_2$-RKKfWFWG-COOH | 1178.6 | 1178.5 | 3.39 | 90% | 8.7 |
| **MFIGAF003** | $NH_2$.RKKpWFWG-COOH | 1104.6 | 1104.6 | 3.42 | 91% | 9.2 |
| **MFIGAF004** | $NH_2$-RKKPWFWG-COOH | 1104.6 | 1104.6 | 3.63 | 83% | 6.1 |

*SYNTHETIC EXAMPLE 5 - Preparation of novel rationally designed cyclic PAF26 derivative peptides*

MFIGAF005, MFIGAF001, MFIGAF007, and MFIGAF008 were synthesised by performing the *N*- to *C*-terminus cyclisation of the peptides MFIGAF000, MFIGAF002, MFIGAF003, and MFIGAF004. Synthesis of peptides MFIGAF000, MFIGAF002, MFIGAF003, and MFIGAF004 were carried same as described previously. Then 1 eq. of side chain protected CMN peptide was dissolved in DMN at a concentration of 100 mg/mL. 1-[bis(dimethylamino)methylene]-1*H*-1,2,3-triazolo[4,5-b]pyridinium-3-oxid hexafluorophosphate (HATU, 1 eq.) and 2.5 eq. DIPEA were added into the solution. The reaction was left to proceed for 2 h at r.t. and then the crude product was precipitated in water. Strong cleavage was then performed using Cleavage cocktail 2 (85 : 5 : 5 : 5 - TFA/water/TIS/Phenol) for 4 h at r.t.. After this, the solution was concentrated and precipitated using $Et_2O$. Finally, the purification was carried out by semi-preparative RP-HPLC *(Agilent semi-prep* C18 column, 21.2 × 150 mm, 10 μm diameter size) with the mobile phase as ACN (0.1% FA)/$H_2O$ (0.1% FA) and a flow rate of 2 mL·min$^{-1}$. Pure fractions were lyophilised after purification to produce the peptides listed in Table 8.

**Table** 8 - Characterisation data of novel designed cyclic peptides

| Compound | Structure | MS (m/z): [M+H]$^+$ | | HPLC $t_R$ (min) | Purity | Amount (mg) |
|---|---|---|---|---|---|---|
| | | Calculated | Found | | | |
| **MFIGAF005** | Cyclo(RKKaWFWG) | 1060.6 | 1060.9 | 3.39 | >99% | 5.7 |
| **MFIGAF001** | Cyclo(RKKfWFWG) | 1136.6 | 1136.9 | 3.62 | >99% | 7.6 |
| **MFIGAF007** | Cyclo(RKKpWFWG) | 1086.6 | 1086.9 | 3.41 | >99% | 11.3 |
| **MFIGAF008** | Cyclo(RKKPWFWG) | 1086.6 | 1086.9 | 3.36 | >99% | 3.8 |
| **MFIGAF001a** | Cyclo(RKKfWFWK(Cy5)G) | 1750.9 | 1751.1 | 4.85 | >99% | 4.1 |
| **MFIGAF001b** | Cyclo(RKKfWFWK(CF)G) | 1624.8 | 1624.1 | 4.18 | 76% | 26.0 |
| **MFIGAF001c** | Cyclo(RKKfWFWK(Cy5)) | 1693.9 | 1693.2 | 4.48 | 93% | 13.5 |
| **MFIGAF001d** | Cyclo(RKKfWFWK(CF)) | 1567.8 | 1567.4 | 4.08 | 91% | 5.6 |

**Table 9** - Spectroscopic characterization data of cyclic peptides

| Compound | Structure | Max absorption wavelength (nm) | Max emission wavelength (nm) |
|---|---|---|---|
| **MFIGAF001a** | Cyclo(RKKfWFWK(Cy5)G) | 630 | 660 |
| **MFIGAF001b** | Cyclo(RKKfWFWK(CF)G) | 498 | 530 |
| **MFIGAF001c** | Cyclo(RKKfWFWK(Cy5)) | 630 | 660 |
| **MFIGAF001d** | Cyclo(RKKfWFWK(CF)) | 498 | 530 |

## IMAGING METHODS AND SAMPLE PREPARATION

### *Sample preparation for live-cell imaging*

The *N. crassa* mat A WT used for confocal live-cell imaging was grown on 100% Vogel's agar medium at 25°C for 3-5 days before the spores were harvested. The A. *fumigatus* CEA10 strain used for confocal live-cell imaging was grown on 100% Vogel's medium at 37°C for 4-5 days before the spores were harvested. Before the confocal experiments were performed, *N. crassa* spores were incubated in eight-well slide culture chambers for 3 h at 25°C in liquid 10% Vogel's medium and A. *fumigatus* spores were incubated for 16 h at 25°C in liquid 10% Vogel's medium in order to allow germination to fully take place for imaging.

### *Wide-field microscopy*

Wide-field epifluorescence microscopy was performed with an inverted Nikon Eclipse TE2000E microscope equipped with DIC optics, a 60 × (1.3 NA) plan fluor objective, and equipped with a Nikon DXM1200F camera and ACT-1 software for image capture. Propidium iodide fluorescence was excited at 550 nm by a LED excitation system (CoolLED, pE300). A 500 nm dichromic filter and a 575 nm long pass emission filter were used for fluorescence visualisation. Images were subsequently processed using ImageJ software. Data was saved in .xlsx format for analysis.

### *Confocal microscopy*

Live-cell imaging of germinated fungi spores was performed using a Leica TCS SP8 confocal laser scanning microscope equipped with photo multiplier tubes (PMT), hybrid GaAsP (HyD) detectors and a 63× water immersion objective. A Leica turntable white light laser (WLL, 450-750nm), argon laser (458nm, 476nm, 488nm and 496nm) and UV laser (405nm) were used for fluorescence excitation.

**Table 10** - Imaging conditions for fluorescently labelled peptides

| Compound | Fluorophore | Ex wavelength (nm) | Em wavelength range (nm) |
|---|---|---|---|
| **MFIGAF0001a**<br>**MFIGAF0001c** | Cy5 | 633 | 650-670 |
| **MFIGAF0001b**<br>**MFIGAF0001d** | Carboxyfluorescein | 498 | 510-550 |

It will be understood that imaging conditions for other fluorophores will be apparent to the skilled person.

### *Image processing*

Images obtained from this study were processed and analyzed using Imaris scientific 3D/4D image processing and analysis software 8.0 developed by Bitplane (Zurich, Switzerland).

### *Biological Examples*

### *Fungal species and strains*

All fungal strains used are listed in Table 11 to 13.

**Table 11** - *N. crassa* strains

| Strain | Mat | Genotype | Locus | Stock No. | Source |
|---|---|---|---|---|---|
| WT | A | *74-OR23-1V* | - | 0013 | FGSC #2489 |

**Table 12** - A. *fumigatus* strains

| Strain | Genotype | Source |
|---|---|---|
| CEA10 | *MAT1-1, wildtype* | FGSC #A1163 |

(continued)

| Strain | Genotype | Source |
|---|---|---|
| SCS03 | *ptrA::PgpdA::sGFP::rab5* | C. Seidel, unpub |
| YFP-*A. fumigatus* | *gpdA::yfp (ptrA)* | A gift from Sven Krappmann (not published) |

**Table 13** - *F. oxysporum* strains

| Strain | Genotype | Locus | Stock No. | Source |
|---|---|---|---|---|
| *F. sp. lycopersici* | *wildtype* | FOXG_12732 | - | FGSC 9935 |

***Culture media and growth conditions***

The recipes of the stock media solutions used in this study are shown in Tables 14 and 15. The stocks were diluted and mixed with agar (Oxoid Ltd.) to 2% (w/v) before autoclaving. The agar was omitted for Vogel's liquid medium.

**Table 14** - Composition of Vogel's 50x stock solution per litre $dH_2O$

| Chemical | MassNolume |
|---|---|
| $Na_3Citrate \cdot 2H_2O$ | 126.70 g |
| $KH_2PO_4$ | 250.00 g |
| $NH_4NO_3$ | 100.00 g |
| $MgSO_4 \cdot 7H_2O$ | 10.00 g |
| $CaCl_2 \cdot 2H_2O$ | 5.00 g |
| Trace Element Solution | 5.00 ml |
| d-Biotin Solution (50 mg / L 50% EtOH) | 5.00 ml |

**Table 15** - Composition of Vogel's trace element solution per litre $dH_2O$

| Chemical | MassNolume |
|---|---|
| $C_6H_8O_7 \cdot 1H_2O$ | 5.00 g |
| $ZnSO_4 \cdot 7H_2O$ | 5.00 g |
| $Fe(NH_4)_2SO_4 \cdot 7H_2O$ | 1.00 g |
| $CuSO_4 \cdot 5H_2O$ | 0.25 g |
| $MnSO_4 \cdot 1H_2O$ | 0.05 g |
| $H_2BO_4$ | 0.05 g |
| $Na_2MoO_4 \cdot 2H_2O$ | 0.05g |
| Note: Ingredients are added in order shown, and stirred until dissolved before the next is added. | |

***Culturing and harvesting Neurospora crassa***

*N. crassa* incubations were carried out at 25°C in an incubator under constant artificial light, unless otherwise specified.

For conidial cultures, the strains were inoculated on small (35 × 10 mm) Vogel's medium plates (Vogel, 1956) and incubated for five days. Conidia were harvested from cultures on day's five to seven. Conidia were not harvested later than this to ensure all conidia were of the same age.

For the mutant strains, the media was supplemented with Hygromycin B (Calbiochem®) to a final concentration of 200 μg/ml by adding 52 μl of Hygromycin B to 300 ml of Vogel's media.

The *N. crassa* conidia were harvested from the plates in sterile $dH_2O$ by washing 1 ml repeatedly over the culture surface. The resulting suspension was then filtered through a triple layer of Miracloth to remove fragments of hyphae. The spore suspension was then vortexed vigorously to separate conidia and ensure an even distribution of them. 10 μl of suspension was mixed into 990 μl $dH_2O$ and the conidial density determined using a Fuchs-Rosenthal haemocytometer (http://www.marienfeld-superior.com).

***Culturing and harvesting Aspergillus fumigatus***

*Aspergillus fumigatus* strains were grown on Sabouraud dextrose agar (SAB agar) at 37°C for 3 days. Conidia were collected in phosphate-buffered saline supplemented with 0.1% tween-20 (PBST) by gently scraping a spatula over the fungal colony surface. The spore solutions were then passed through autoclaved Miracloth to remove any hyphal residues and stored at 4°C. The spores were counted and the concentrations was determined in the same was as described for *N. crassa.*

***Culturing and harvesting Fusarium oxysporum***

*F. oxysporum* was grown in liquid potato dextrose broth (PDB) at 28 °C with shaking (170 rpm) for 5 days. The suspension was filtered through folded Miracloth (4 layers) and centrifuged for 10 min at RT. The supernatant was discard and the pellet was resuspend in 1.5 mL of sterile $dH_2O$ and quantified.

***Culturing and harvesting of Candida and Cryptococcus strains***

*Candida* strains used were grown on SAB agar at 37 °C for 3 days, and the *Cryptococcus* strain was grown on SAB agar at at 37 °C for 6 days. Cells were harvest using sterile inoculation loop by taking a single colony and resuspending in phosphate-buffered saline supplemented with 0.1% tween-20 (PBST). The concentration of cells was then quantified with a haemocytometer. For the determination of the minimum inhibitory concentration, cell density was adjusted to $10^6$ cells/mL with 20% liquid Vogel's medium.

***Culturing and harvesting of bacteria strains***

Bacteria strains used were grown on Lysogeny Broth (LB) agar at 37 °C for 1 day, and were harvest using sterile inoculation loop by taking a single colony and resuspending in PBST. The concentration of cells was quantified using a haemocytometer. For the determination of the minimum inhibitory concentration, cell density was adjusted to $10^6$ cells/mL with 20% liquid LB medium.

*BIOLOGICAL EXAMPLE 1 - Antifungal activity determination*

***$IC_{50}$ determinations based on biomass optical density measurements***

Dose dependent inhibition of fungal growth by all non-labelled peptides (indicated by $IC_{50}$ values) was determined via this methodology. The assays were performed using Nunc 262162 U96 polystyrene round bottomed clear plates (www.nuncbrand.com).

When testing the peptides against A. *fumigatus,* peptides at different concentrations were mixed with A. *fumigatus* conidia to reach a final volume of 100 μL per well. The final conidial concentration was $5 \times 10^5$ cells/mL in 10% Vogel's medium. After 24 h of incubation at 37°C in 96 well plates, fungal growth with the different peptide concentrations was determined by measurement of the optical density at 610 nm in a TriStar LB 941 Multimode Microplate Reader. The $IC_{50}$ values were determined using four parameter logistic regression formula provided by Sigma Plot 10.0. Values are represented as means ± SEM from two independent experiments (n = 3).

When testing the peptides against *N. crassa* and *F. oxysporum,* experiments were carried as described above apart from the incubation temperature was changed to 25°C for *N. crassa* and 35°C for *F. oxysporum.*

***$IC_{50}$ determination based on a luciferase assay for fluorescently labelled compounds***

The $IC_{50}$ of the fluorescently labelled peptides cannot be accurately determined using the optical density method described in the last section. This is because the fluorophore structures absorbed light at 610 nm which was the wavelength used for the optical density measurements. In order to overcome this, the BacTiter-Glo™ Microbial Cell Viability Assay kit purchased from Promega was used. The BacTiter-Glo™ Reagent relies on the properties of a proprietary thermostable luciferase (Ultra-Glo™ Recombinant Luciferase) and a proprietary formulation for extracting ATP from fungal cells which will generates a 'glow-type' luminescent signal. This is produced by the luciferase reaction shown in Scheme 1, which has a signal half-life of over 30 min depending on the experimental conditions. The luminescent signal is proportional to the amount of ATP present, which is directly proportional to the number of living cells in culture.

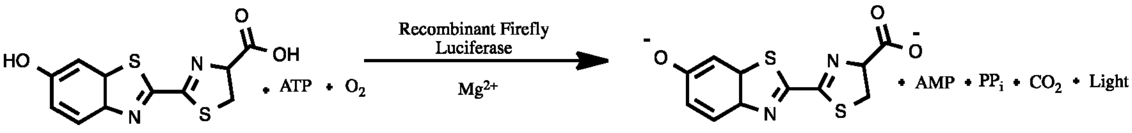


Beetle Luciferin

Oxyfuciferin

**Scheme 1** - The luciferase reaction. Mono-oxygenation of luciferin is catalyzed by luciferase in the presence of $Mg^{2+}$, ATP and molecular oxygen.

The $IC_{50}$ assays for all fluorescently labelled peptides were performed using Greiner CELLSTAR white polystyrene wells flat bottomed 96 well plates. The incubation conditions remained the same for the optical density measurement described in the previous section. Once the incubation procedure was finished, the plate was equilibrated to room temperature. A mixture of BacTiter-Glo™ buffer and substrate was then added to the plate (100 μL per well). The plate was shaken for 5 min and read using the Tristar LB491 luminometer. Data analysis was performed using Sigma Plot 10.0. All values shown are presented as means ± SEM from two independent experiments (n = 3).

**Table 16** - Antifungal efficacy determined for MFIGAF001 and analogues

| Compounds | ***Aspergillus fumigatus*** **(37°C, 24 h)** | ***Fusarium oxysporum*** **(35°C, 24 h)** |
|---|---|---|
| | **$IC_{50}$ (mg/L)*, Conidia (10% Vogel's)** | |
| **PAF26** | 8.0 | 7.2 |
| **MFIGAF000** | 5.3 | 4.2 |
| **MFIGAF001** | 1.4 | 1.9 |
| **MFIGAF002** | 2.8 | 3.5 |
| **MFIGAF003** | 5.2 | 4.1 |
| **MFIGAF004** | 5.2 | 4.1 |
| **MFIGAF005** | 6.3 | 3.1 |
| **MFIGAF007** | 5.2 | 3.4 |
| **MFIGAF008** | 5.5 | 3.8 |
| **MFIGAF001a** | 1.3 | 1.5 |
| **MFIGAF001b** | 2.1 | 2.3 |
| **MFIGAF001c** | 1.5 | 1.8 |
| **MFIGAF001d** | 2.9 | 3.0 |
| | **$IC_{99}$ (mg/L), Conidia (10% Vogel's)** | |
| **MFIGAF001** | 5.7 | - |
| | **$IC_{99}$ (mg/L), Conidia (RPMI)** | |
| **Voriconazole** | 1 | - |
| **Natamycin** | 39 | - |
| *$IC_{50}$ (mg/L) values represent averages of at least three independent experiments | | |

<u>*BIOLOGICAL EXAMPLE 2*</u>

Antifungal activities have been evaluated for MFIGAF001 against various fungi, including *Aspergillus fumigatus* and *Fusarium oxysporum,* indicating that MFIGAF001 is exhibiting a sub-micromolar $IC_{50}$ value against major pathogenic fungi. Antibacterial activity against *Staphylococcus aureus* was also observed.

**Table 17**

| Strains | Tested concentration (μM) | Medium | MFIGAF001 MIC (μM) |
|---|---|---|---|
| ***S. aureus*** | $5x10^5$ | 10% LB | 15.6 |
| ***A. fumigatus*** **(MFIG001)** | $5x10^5$ | 10% Vogel's | 5 |
| ***F. oxysporum*** | $5x10^5$ | 10% Vogel's | 6.25 |
| ***C. inconspicua*** **(ATCC 16783)** | $5x10^5$ | 10% Vogel's | 2 |

(continued)

| Strains | Tested concentration (μM) | Medium | MFIGAF001 MIC (μM) |
|---|---|---|---|
| ***C. auris*** | $5x10^5$ | 10% Vogel's | 2 |
| ***C. albican*** | $5x10^5$ | 10% Vogel's | 2 |
| ***C. glabrata* (NCPF 3309)** | $5x10^5$ | 10% Vogel's | 3.9 |
| ***C. krusei* (ATCC 6258)** | $5x10^5$ | 10% Vogel's | 3.9 |
| MIC = minimum inhibitory concentration | | | |

*BIOLOGICAL EXAMPLE 3*

MFIGAF001 exhibits great stability after 24 h treatment at 37°C (99% purity, that is, intact peptide remaining) with *Streptomyces griseus* protease cocktail, compared to its analogue compound (0% purity) **(Figure 1).** Therefore, MFIGAF001 has good thermal stability and is resistant to protease digestion.

**Table 18** - Thermal stability over time with *Streptomyces griseus* protease

| Time (h) | Purity ( % of intact pepti de left) | | |
|---|---|---|---|
| | **PAF26** | **MFIGAF001** | **MFIGAF008** |
| 0 | 94% | 99% | 99% |
| 1 | 70% | 99% | 99% |
| 8 | 8% | 99% | 99% |
| 24 | 0% | 99% | 99% |

*BIOLOGICAL EXAMPLE 4*

MFIGAF001 exhibit virtually no cytotoxicity even at 0.5 mg/L (440 μM) against A549 **(Figure 2).**

Cytotoxicity of MFIGAF001 against human lung epithelia cell line A549 was determined using Vybrant MTT Cell Proliferation Assay Kit from Invitrogen.

*BIOLOGICAL EXAMPLE 5*

Cell viability assays have also been performed on Human Red Blood Cells (HRBCs), indicating that MFIGAF001/MFIGAF002/ MFIGAF001a (10 μM) have no haemolytic activity against HRBCs **(Figure 3).**

Erythrocytes were isolated from freshly drawn, anticoagulated human blood and diluted in PBS (1:5). An amount of 50 μl of erythrocyte suspension was added to 50 μl of compounds at 10 μM. DMSO was used as positive control and PBS as negative control. The plate was incubated at 37 °C for 1 h, each well was diluted with 150 μl of PBS and the plate was centrifuged at 1,200g for 15 min. A total of 100 μl of the supernatant from each well was transferred to a fresh plate, and the absorbance at 350 nm was measured in a microplate reader. Data is represented as % of cell viability as means from three independent experiments with n=3.

*BIOLOGICAL EXAMPLE 6*

MFIGAF001, MFIGAF001a and MFIGAF001b exhibit no obvious toxicity **(Figure 4).**

MFIGAF001 was also assessed independently by a CRO (Evotec) in an OECD compliant reconstructed cornea-like epithelial cell model (RhCE, EpiOcularTM). No cytotoxicity was observed, even at 0.5 mg/L (440 μM).

**Table 19** - Mean tissues OD and Viabilities

| | Mean of OD | Mean of Viabilities (%) | Diff. of Viabilities | Classification | |
|---|---|---|---|---|---|
| **Negative Control** | 2.89 | 100.0 | 8.41 | NI | qualified |
| **Positive Control** | 0.581 | 26.5 | 5.66 | I | qualified |
| **MFIGAF001 0.1 mg/mL** | 1.910 | 87.3 | 0.27 | NI | qualified |
| **MFIGAF001 0.2 mg/mL** | 1.908 | 87.2 | 4.34 | NI | qualified |
| **MFIGAF001 0.4 mg/mL** | 1.940 | 88.6 | 4.45 | NI | qualified |

(continued)

| | Mean of OD | Mean of Viabilities (%) | Diff. of Viabilities | Classification | |
|---|---|---|---|---|---|
| **MFIGAF001 0.5 mg/mL** | 2.051 | 93.7 | 2.31 | NI | qualified |
| OD = optical density; NI = Not irritant; I = Irritant | | | | | |

**Table 20** - Mean tissues OD and Viabilities

| | Mean of OD | Mean of Viabilities (%) | Diff. of Viabilities | Classification | |
|---|---|---|---|---|---|
| **Negative Control** | 3.350 | 100.0 | 1.26 | NI | OD NC ≥ 2.5 |
| **Positive Control** | 1.022 | 30.5 | 13.57 | I | OD NC ≥ 2.5 |
| **MFIGAF001a (10 μM)** | 3.282 | 98.0 | 0.83 | NI | OD NC ≥ 2.5 |
| **MFIGAF001b (10 μM)** | 3.319 | 99.1 | 1.41 | NI | OD NC ≥ 2.5 |
| OD = optical density; NI = Not irritant; I = Irritant | | | | | |

*BIOLOGICAL EXAMPLE 7*

Tolerability studies were performed on rabbit eyes *in vivo* and no eye irritation occurred **(Figure 5).** Three rabbits (rabbits 1, 2 and 3) were tested. All rabbits appeared unaffected immediately post-treatment and through the next 7 days with no signs of irritation and no change in general condition. MFIGAF001a was used at 10 μM and 50 μL and was admitted into the right eye of the rabbits.

MFIGAF001a is well tolerated in rabbit eye.

*BIOLOGICAL EXAMPLE 8*

**Porcine Cornea preparation**

Freshly harvested porcine eyes (whole globe) were collected from a local abattoir within 24 hours of sacrifice. All subsequent procedures were performed under aseptic conditions. The eyeballs were washed with 50 mL of sterile phosphate-buffered saline supplemented with 0.1% tween-20 and 5% penicillin-streptomycin (PBST-PS, Sigma-Aldrich) for 10 minutes. Corneal dissection was carried out in Class II microbiological safety cabinet to avoid potential contamination. Using sterile instruments, the cornea was removed from the globe using corneo-sceral dissection at the limbus, leaving an 2-3 mm scleral rim. Any other tissue such as iris or lens was then removed, leaving the corneal button alone. The cornea was then gently washed 3 times in 5 mL sterile PBST-PS by gentle submergence and shaking for approx. 1 minute. Sterile tweezers were employed for holding the scleral rim, and taking care not to scratch the corneal surface during the procedure. Washed corneas were then placed in sterile petri-dishes containing fresh PBST-PS until completion of the dissection session (< 1 hour). Upon completing dissection, each individual corneal button was transferred into a well of a 6-well tissue culture plates containing 1 mL of culturing medium (either RPMI-PS or PBST-PS, Table 1) and one cornea button with epithelium facing up.

**Infection of Corneas with *Aspergillus fumigatus***

YFP-*A. fumigatus* spores were harvested from 3 days old cultures grown on SAB agar at 37 °C in PBST and quantified using a haemocytometer.

The lidded 6-well culturing plate containing cornea buttons and medium was placed in a Class II cabinet, the central region of corneal epithelium was lightly scratched using sterile hypodermic needle (gauge 27G, B Braun™, Fisher Scientific) to create 5 linear abrasions in parallel. 1 μL sterile inoculation loops were used to transfer 1 μL of inoculum concentrated from $10^6$ to $10^3$ spore/mL (containing c.~1000 to c.~1 spores dependent upon experimental setup) in a stroking motion. Viable counts for confirming the number of spores in each suspension were performed at the time of each inoculation. The 6-well culture plate was closed and allowed to sit for 10-15 minutes prior to transferring to incubator. The corneal buttons were incubated for desired periods (from 1 hour to 48 hours dependent upon experimental setup) at 37 °C, supplemented with 5% $CO_2$. Uninfected corneas were scratched and inoculated with 1 μL of sterile PBS in the same way, and then maintained along with the infected corneas in the same 6-well culture plate for each experiment. The culture medium was refreshed every 24 hours during the culturing period, fresh medium was added by pipetting avoiding the cornea after removal of existing medium . Regular checks for the presence of fungus in the medium in which each cornea was placed were performed every 24 hours to ensure that the only infection site was on the top sode of the corneal tissue

and not from the beneath. Corneal samples with macroscopic visible fungal growth in the medium were removed from the experiment. After desired culturing period, the corneal buttons were removed from the incubator for subsequent imaging. When multiple cornea buttons were waiting to be imaged, the unimaged ones were stored at 4 °C after wrapping the 6-well culture plate in Parafilm (Bemis, Heathrow Scientific) for a short period (less then 2 hours) while the other ones were imaged.

The progression of infection was monitored by live cell imaging at desired time points using confocal microscopy. Cultured cornea buttons were removed from the 6-well culture plate and placed with epithelium facing down into 2-well ibidi imaging chambers (ibidi GmbH, Martinsried, Germany). The imaging chamber was filled with 1 mL of medium matching that used for the infection experiments (either RPMI-PS or PBST-PS, Table 1) . A Leica TCS SP8 confocal microscope (Leica Microsystems Ltd., Milton Keynes, UK) with long working distance 25x water immersion objective was employed for imaging the development of A. fumigatus corneal infection. Excitation wavelength at 514 nm and emission wavelength between 525-545 nm were used for imaging the YFP expressed by A. fumigatus. The 'Z-compensation function' was used for fine adjustment of the laser excitation to compensate the signal reduction in the deeper section of the cornea. These adjustments were done across 5 points of the z-stack, preventing over or under-exposure of acquisition. Acquired images were analysed using Imaris v8.0 software (Bitplane Scientific software module; Zurich, Switzerland). The 'surface module' on Imaris was used to translate the fluorescent signal into a 'surface model' for quantification of occupied live fungal biomass.

MFIGAF001 reduced fungal burden in *ex vivo* cornea infected with YFP-*A. fumigatus.* On a macroscopic level, **Figure 6** shows the fungal infection had greatly progressed in saline treated cornea 1, while the MFIGAF001 treated cornea 2 showed less severe infection after the 72h of treatment.

Two corneas were infected with ~1 spore/cornea YFP expressing A. *fumigatus,* yellow fluorescent signal indicates the live fungal burden that penetrated into the corneal stroma. Infections were established after 36 hours incubation in PBST-PS. Corneas were treated with saline or MFIGAF001 by topical administration after the establishment of infection. All cornea were treated with one drop of reagent every 2 hours for the first 8 hours, then one drop every 8 hours for the remaining 16 hours. Treatment was continued for 72 hours with this treatment regimen. Natamycin was used at 120mg/L.

**Figure 7** shows the elimination of live fungal burden from the MFIGAF001 treated cornea 2, where the fluorescent signal (yellow in the original graphs, white reproduced here) indicates the live fungal burden that penetrated into the corneal tissue.

*BIOLOGICAL EXAMPLE 9*

MFIGAF001 exhibited comparable efficacy against Natamycin *ex vivo.* All corneas were treated with one drop every 2 hours for the first 8 hours, then one drop every 8 hours for the remaining 16 hours. Treatment was continued for 72 hours with this treatment regimen. MFIGAF001 was used at 100 mg/L. Natamycin was used at 120 mg/L. The results are shown in **Figure 8,** where the fluorescent signal (yellow in the original graphs, white reproduced here) indicates the live fungal burden that penetrated into the corneal tissue.

*BIOLOGICAL EXAMPLE 10*

The inventors have shown that the fluorescent diagnostics MFIGAF001a and MFIGAF001b label all major pathogenic fungi that cause fungal keratitis, even at low concentrations. The major pathogenic fungi include *C. albicans, F.oxysporum, A. fumigatus,* and C. *neoformans.* **Figure 9** shows live cell imaging of MFIGAF001a and MFIGAF001b (fluorescent, 2.5 μM) after 15 min incubation at 37°C in RPMI with various pathogenic fungi (C. *albicans, F.oxysporum, A. fumigatus,* and *C. neoformans*), demonstrating that MFIGAF001a exhibits rapid and highly fluorogenic labelling. **Figure 10** shows live cell imaging of MFIGAF001a (fluorescent, 2.5 μM) incubated at 37°C in RPMI with A. *fumigatus.*

*BIOLOGICAL EXAMPLE 11*

MFIGAF001 selectively labels A. *fumigatus* over human epithelial cell line A549. **Figure 11** shows live cell imaging of A. *fumigatus* in co-cultures with human epithelial cell line A549, 10 min after addition of MFIGAF001a (fluorescent, 2.5 μM) at 37°C in RPMI.

*BIOLOGICAL EXAMPLE 12*

MFIGAF001a in a pig corneal infection model was investigated **(Figure 12).** Live cell imaging of MFIGAF001a (red in the original data, shown in white here; conditions: 2.5 μM, 15 min incubation at 37°C) selectively labelled A. *fumigatus.*

Porcine corneal was infected with A. *fumigatus* using the same method described above **(Biological Example 8).** After confirming the success of infection, 1 drop of MFIGAF001 solution in water (2.5μM) was added topically to the corneal surface and incubated for 15 min at 37°C before confocal microscopy.

## BIOLOGICAL EXAMPLE 13

MFIGAF001a exhibits great selectivity over bacteria such as gram negative bacteria *Pseudomonas* **(Figure 13).** MFIGAF001a labels gram positive bacteria *Staphylococcus aureus.* **(Figure 14).**

## BIOLOGICAL EXAMPLE 14

The inventors have demonstrated that MFIGAF001a can be used to differentiate fungal keratitis and gram positive bacterial keratitis.

**Figure 15** shows live cell imaging of MFIGAF001a (fluorescent, 2.5 μM, 15 min incubation at 37°C) in a pig corneal infection model co-infected by A. *fumigatus* and S. *aureus* for 48 h in Roswell Park Memorial Institute (RPMI) 1640 Medium (purchased from Thermo Fisher Scientific). The labelled compound can be used to distinguish fungus from S. *aureus* when using confocal microscopy based on assessing the morphology of the areas of fluorescence. The fungal hyphea can be observed and identified based on its characteristic filamentous shape, and its much larger size compared to the bacteria which appears to be much smaller and having a dotted shape.

## References

A number of publications are cited above in order to more fully describe and disclose the invention and the state of the art to which the invention pertains. Full citations for these references are provided below.

Arikan et al., 2001, In vitro susceptibility testing methods for caspofungin against Aspergillus and Fusarium isolates, Antimicrob. Agents Chemother, 45, 327-330.

Bacon et al., 1993, Acanthamoeba keratitis. The value of early diagnosis., Ophth, 100(8), 1238-43.

Brown et al., 2021, The Global Incidence and Diagnosis of Fungal Keratitis, Lancet Infect. Dis., 21(3), e49-e57.

Burton et al., 2011, Microbial keratitis in East Africa: why are the outcomes so poor? Ophthalmic Epidemiol., 18(4), 158-63.

Chidambaram et al., 2016, Prospective Study of the Diagnostic Accuracy of the In Vivo Laser Scanning Confocal Microscope for Severe Microbial Keratitis, Ophthalmology, 123(11), 2285-93.

Chidambaram et al., 2018, Epidemiology, risk factors, and clinical outcomes in severe microbial keratitis in South India, Ophthalmic Epidemiol., 1-9.

Dalmon et al., 2012, The clinical differentiation of bacterial and fungal keratitis: a photographic survey. Invest. Ophthalmol. Vis. Sci., 53(4), 1787-91.

Delano, 2012, The PyMOL Molecular Graphics System, DeLano Scientific LLC: San Carlos, CA. EP 2846840 A2

Fasman, 1989, Molar absorptivity and values for proteins at selected wavelength of the ultraviolent and visible region, In Practical Handbook of Biochemistry and Molecular Biology, 196-327.

Grosdidier et al., 2011, SwissDock, a protein-small molecule docking web service based on EADock DSS, Nucl. Acids Res., 39 (Web Server Issue), W270-277.

Lee et al., 2018, Exp. Eye Res., 174, 51-58.

Mehio et al., 2010, Identification of protein binding surfaces using surface triplet propensities, Bioinformatics, 26, 2549-2555.

Nelson et al., 2004, Calcium measurement in living filamentous fungi expressing codon-optimized aequorin, Mol. Microbiol., 52, 1437-50.

Neoh et al., 2014, Clinical utility of caspofungin eye drops in fungal keratitis, Int. J. Antimicrob. Agents, 44(2), 96-104.

Oldenburg et al., 2017, Evolution of practice patterns for the treatment of fungal keratitis, JAMA Ophthalmol, 135(12), 1448-1449.

Perlin et al., 2018, Med. Mycol., 56(7), 796-802.

Pettersen et al., 2004, UCSF Chimera--a visualization system for exploratory research and analysis, J. Comput. Chem., 25, 1605-12.

Prajna et al., 2013, The mycotic ulcer treatment trial: a randomized trial comparing natamycin vs voriconazole, JAMA Ophthalmol., 131(4), 422-9.

Tuft et al., 2013, Microbial Keratitis. Focus: Royal College of Ophthalmologists, UK.

Vogel, 1956, A convenient growth medium for Neurospora (Medium N), Microbial Genet. Bull., 13, 42-43.

For standard molecular biology techniques, see Sambrook, J., Russel, D.W. Molecular Cloning, A Laboratory

Manual. 3 ed. 2001, Cold Spring Harbor, New York: Cold Spring Harbor Laboratory Press.

**Claims**

1. A compound of Formula I or Formula II:

Cyclo(Arg-Lys-Lys-Xaa-Trp-Phe-Trp-Yaa) **Formula I**

$R^1$-Arg-Lys-Lys-Xaa-Trp-Phe-Trp-Yaa-$R^2$ **Formula II**

or a pharmaceutically acceptable salt thereof, wherein

$R^1$ is H, $C_{1-4}$ alkyl, $C_{1-4}$ acyl, phosphoryl ($PO_3^{2-}$), or biotinyl;
$R^2$ is OH, $NH_2$, H or O($C_{1-4}$ alkyl);
Xaa is a natural or unnatural amino acid residue having a hydrophobic side chain;
Yaa is selected from Gly, Lys, Lys-Gly, Lys*, Lys*-Gly , Zaa, Zaa*, Zaa-Gly, and Zaa*-Gly, where Zaa is an unnatural amino acid having a free amine, azide or alkyne side chain, and Zaa* is said unnatural amino acid functionalised with or bearing a fluorophore; and
wherein Lys*, if present, is a lysine residue bearing a fluorophore.

2. The compound or pharmaceutically acceptable salt according to claim 1, wherein Xaa is selected from Phe, phe, Ala, ala, Pro, pro, Gly, Val, val, Leu, leu, Ile, ile, Met, met, Tyr, tyr, Trp, and trp; optionally wherein Xaa is selected from phe, ala, pro and Pro.

3. The compound or pharmaceutically acceptable salt according to claim 1 or claim 2, wherein Yaa is Gly.

4. The compound or pharmaceutically acceptable salt according to claim 1 or claim 2, wherein Yaa is Lys * or Lys*-Gly.

5. The compound or pharmaceutically acceptable salt according to claim 4, wherein the compound or salt is of Formula I.

6. The compound or pharmaceutically acceptable salt according to claim 4 or claim 5, wherein the fluorophore is selected from Carboxyfluorescein acid and Cyanine5.

7. The compound or pharmaceutically acceptable salt according to claim 1, wherein the compound is selected from MFIGAF001, MFIGAF005, MFIGAF007, and MFIGAF008:

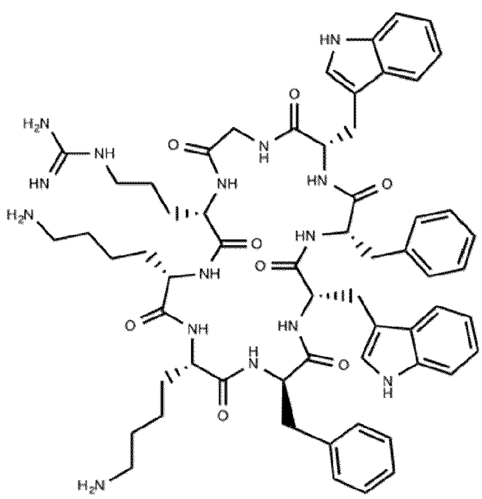

MFIGAF001

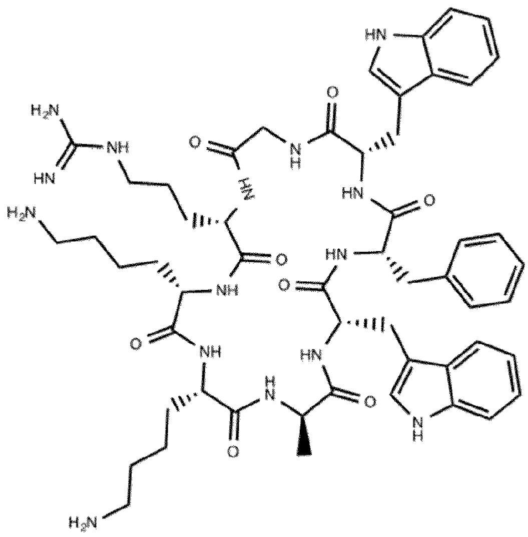

MFIGAF005

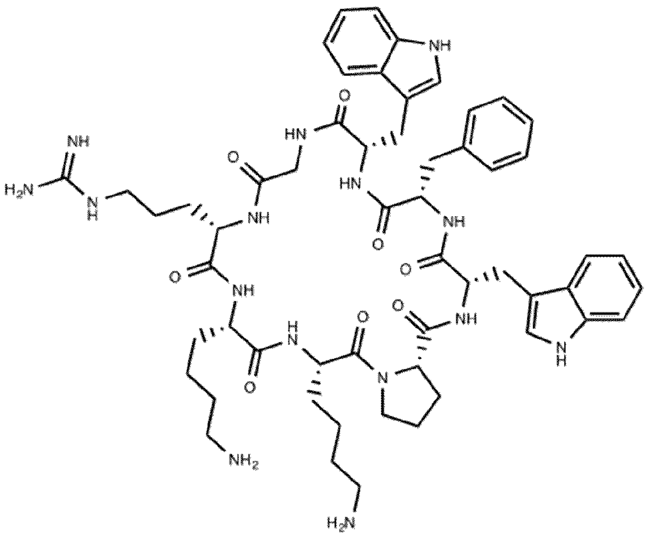

MFIGAF007

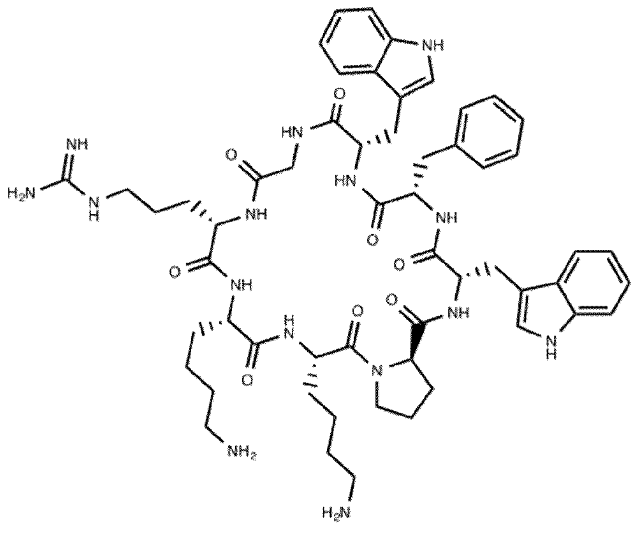

MFIGAF008.

**8.** The compound or pharmaceutically acceptable salt according to claim 1, wherein the compound is fluorophore-labelled MFIGAF001, wherein MFIGAF001 is:

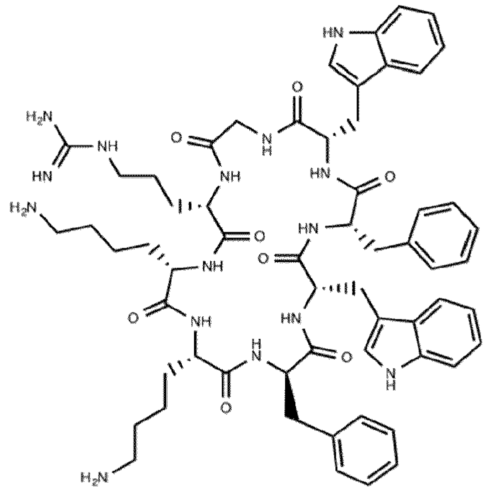

MFIGAF001.

**9.** The compound or pharmaceutically acceptable salt according to claim 1, wherein the compound is selected from MFIGAF000, MFIGAF002, MFIGAF003, and MFIGAF004

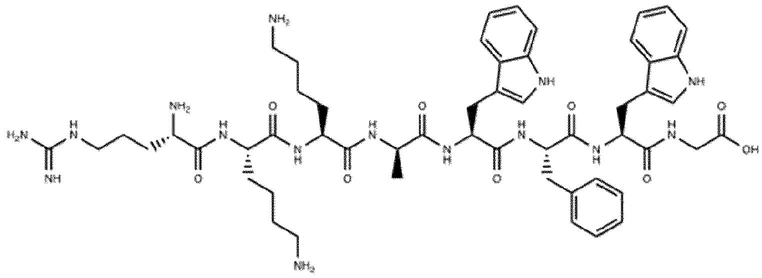

MFIGAF000

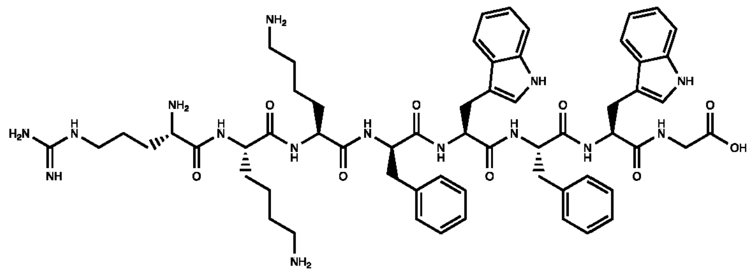

MFIGAF002

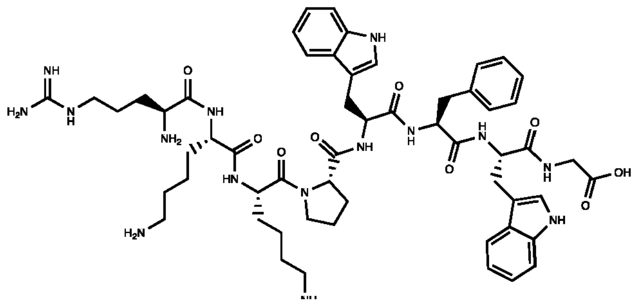

MFIGAF003

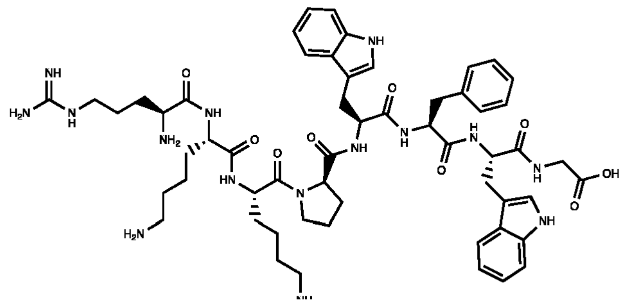

MFIGAF004.

**10.** A pharmaceutical composition comprising the compound or salt of any one of claims 1 to 9 and a pharmaceutically acceptable excipient.

**11.** A compound or salt according any one of claims 1 to 9, or a pharmaceutical composition according to claim 10, for use in a method of treatment.

**12.** A compound or salt according any one of claims 1 to 9, or a pharmaceutical composition according to claim 10, for use in a method of treatment of a fungal infection, a bacterial infection, or an amoebic infection.

**13.** The compound or salt for use according to claim 12, wherein the infection is fungal keratitis or bacterial keratitis.

**14.** A compound or salt according to any one of claims 1 to 9, or a pharmaceutical composition of claim 10, wherein Yaa is Lys*, Lys*-Gly, Zaa*, or Zaa*-Gly, for use in a method of diagnosing a fungal, bacterial or ameobic infection in the eye of a patient, the method comprising:

a) contacting the compound, salt or composition with the patient’s eye;
b) inspecting the patient’s eye to determine if fluorescence is detectable; and
c) noting the fluorescence, if present, and diagnosing the patient with a fungal, bacterial or amoebic infection.

**15.** A method of imaging microbial growth in tissue using a compound or salt according to any one of claims 1 to 9, or a pharmaceutical composition of claim 10, wherein Yaa is Lys*, Lys*-Gly, Zaa*, or Zaa*-Gly, the method comprising:

a) contacting the compound or composition with the tissue;
b) detecting fluorescence of the compound or composition.

**Patentansprüche**

**1.** Verbindung der Formel I oder Formel II:

Cyclo(Arg-Lys-Lys-Xaa-Trp-Phe-Trp-Yaa) **Formel I**

$R^1$-Arg-Lys-Lys-Xaa-Trp-Phe-Trp-Yaa-$R^2$ **Formel II**

oder pharmazeutisch unbedenkliches Salz davon, wobei

$R^1$ H, $C_{1-4}$-Alkyl, $C_{1-4}$-Acyl, Phosphoryl ($PO_3^{2-}$) oder Biotinyl ist;
$R^2$ OH, NH2, H oder O($C_{1-4}$-Alkyl) ist;
Xaa ein natürlicher oder unnatürlicher Aminosäurerest mit einer hydrophoben Seitenkette ist;
Yaa ausgewählt ist aus Gly, Lys, Lys-Gly, Lys*, Lys*-Gly, Zaa, Zaa*, Zaa-Gly und Zaa*-Gly, wobei Zaa eine unnatürliche Aminosäure mit einer freien Amin-, Azid- oder Alkin-Seitenkette ist und Zaa* die unnatürliche Aminosäure ist, die mit einem Fluorophor funktionalisiert ist oder einen Fluorophor trägt; und
wobei Lys*, falls vorhanden, ein Lysin-Rest ist, der einen Fluorophor trägt.

2. Verbindung oder pharmazeutisch unbedenkliches Salz nach Anspruch 1, wobei Xaa ausgewählt ist aus Phe, phe, Ala, ala, Pro, pro, Gly, Val, val, Leu, leu, Ile, ile, Met, met, Tyr, tyr, Trp und trp; optional wobei Xaa ausgewählt ist aus phe, ala, pro und Pro.

3. Verbindung oder pharmazeutisch unbedenkliches Salz nach Anspruch 1 oder Anspruch 2, wobei Yaa Gly ist.

4. Verbindung oder pharmazeutisch unbedenkliches Salz nach Anspruch 1 oder Anspruch 2, wobei Yaa Lys* oder Lys*-Gly ist.

5. Verbindung oder pharmazeutisch unbedenkliches Salz nach Anspruch 4, wobei die Verbindung oder das Salz der Formel I entspricht.

6. Verbindung oder pharmazeutisch unbedenkliches Salz nach Anspruch 4 oder Anspruch 5, wobei der Fluorophor aus Carboxyfluoresceinsäure und Cyanin 5 ausgewählt ist.

7. Verbindung oder pharmazeutisch unbedenkliches Salz nach Anspruch 1, wobei die Verbindung ausgewählt ist aus MFIGAF001, MFIGAF005, MFIGAF007 und MFIGAF008:

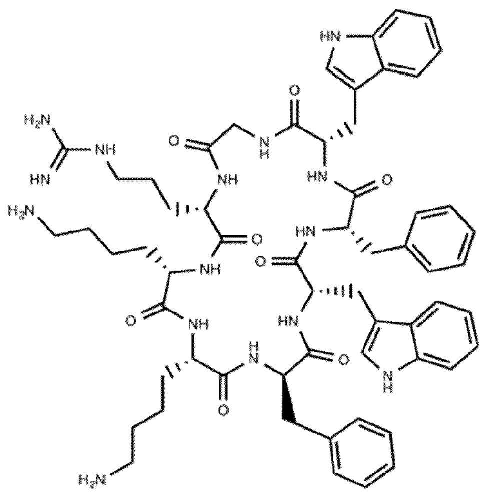

MFIGAF001

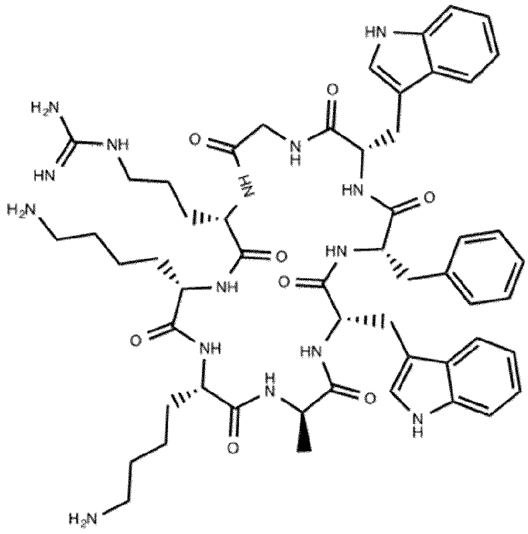

MFIGAF005

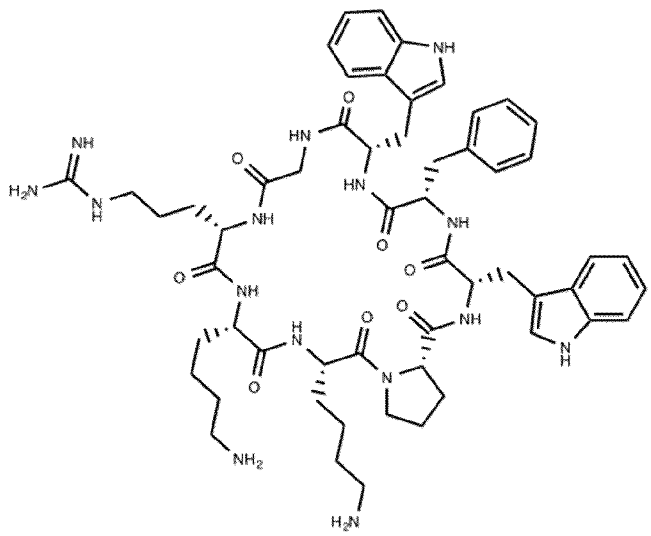

MFIGAF007

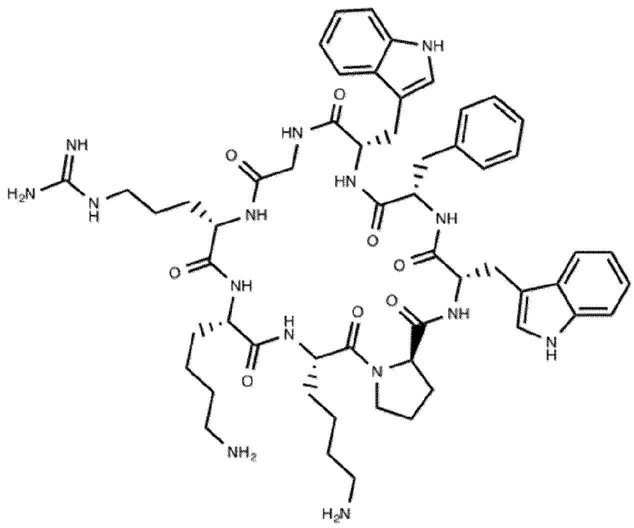

MFIGAF008.

8. Verbindung oder pharmazeutisch unbedenkliches Salz nach Anspruch 1, wobei die Verbindung Fluorophor-markiertes MFIGAF001 ist, wobei MFIGAF001 Folgendes ist:

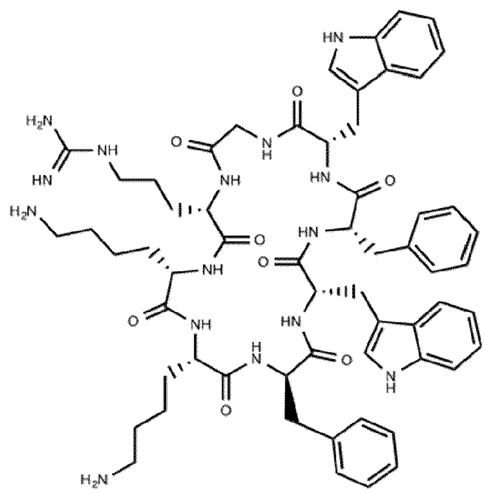

MFIGAF001.

9. Verbindung oder pharmazeutisch unbedenkliches Salz nach Anspruch 1, wobei die Verbindung ausgewählt ist aus MFIGAF000, MFIGAF002, MFIGAF003 und MFIGAF004

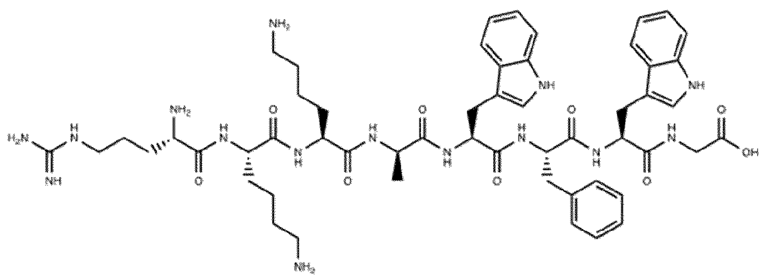

MFIGAF000

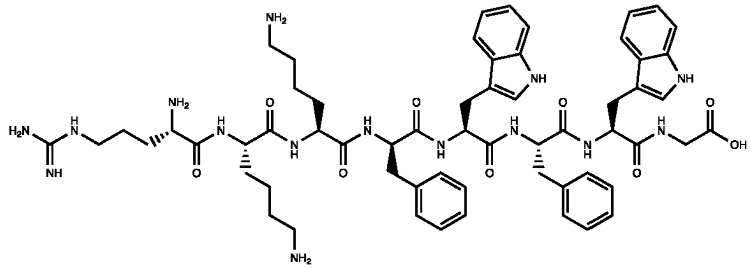

MFIGAF002

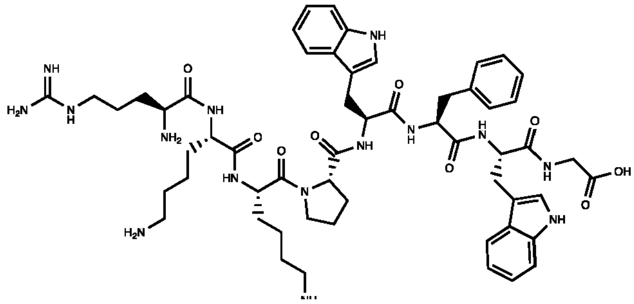

MFIGAF003

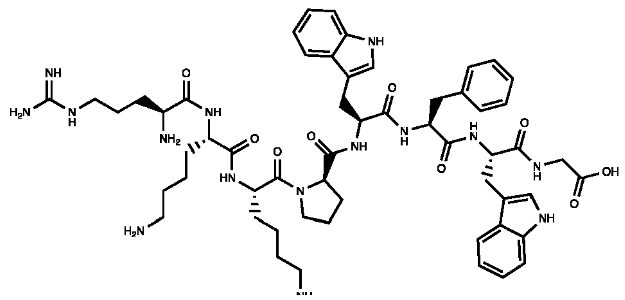

MFIGAF004.

**10.** Pharmazeutische Zusammensetzung, umfassend die Verbindung oder das Salz nach einem der Ansprüche 1 bis 9 und einen pharmazeutisch unbedenklichen Hilfsstoff.

**11.** Verbindung oder Salz nach einem der Ansprüche 1 bis 9 oder pharmazeutische Zusammensetzung nach Anspruch 10 zur Verwendung bei einem Behandlungsverfahren.

**12.** Verbindung oder Salz nach einem der Ansprüche 1 bis 9 oder pharmazeutische Zusammensetzung nach Anspruch 10 zur Verwendung bei einem Verfahren zur Behandlung einer Pilzinfektion, einer bakteriellen Infektion oder einer Amöbeninfektion.

**13.** Verbindung oder Salz zur Verwendung nach Anspruch 12, wobei es sich bei der Infektion um Pilzkeratitis oder bakterielle Keratitis handelt.

**14.** Verbindung oder Salz nach einem der Ansprüche 1 bis 9 oder pharmazeutische Zusammensetzung nach Anspruch 10, wobei Yaa Lys*, Lys*-Gly, Zaa* oder Zaa*-Gly ist, zur Verwendung bei einem Verfahren zum Diagnostizieren einer Pilz-, Bakterien- oder Amöbeninfektion im Auge eines Patienten, wobei das Verfahren Folgendes umfasst:

a) Inkontaktbringen der Verbindung, des Salzes oder der Zusammensetzung mit dem Auge des Patienten;
b) Untersuchen des Auges des Patienten, um zu bestimmen, ob Fluoreszenz nachweisbar ist; und
c) Feststellen der Fluoreszenz, falls vorhanden, und Diagnostizieren einer Pilz-, Bakterien- oder Amöbeninfektion bei dem Patienten.

**15.** Verfahren zum Abbilden von mikrobiellem Wachstum in Gewebe unter Verwendung einer Verbindung oder eines Salzes nach einem der Ansprüche 1 bis 9 oder einer pharmazeutischen Zusammensetzung nach Anspruch 10, wobei Yaa Lys*, Lys*-Gly, Zaa * oder Zaa*-Gly ist, wobei das Verfahren Folgendes umfasst:

a) Inkontaktbringen der Verbindung oder Zusammensetzung mit dem Gewebe;
b) Nachweisen von Fluoreszenz der Verbindung oder Zusammensetzung.

**Revendications**

**1.** Composé de Formule I ou de Formule II :

Cyclo(Arg-Lys-Lys-Xaa-Trp-Phe-Trp-Yaa) **Formule I**

$R^1$-Arg-Lys-Lys-Xaa-Trp-Phe-Trp-Yaa-$R^2$ **Formule II**

ou un sel acceptable pharmaceutiquement de celui-ci, dans lequel

$R^1$ est H, un alkyle en $C_{1-4}$, un acyle en $C_{1-4}$, un phosphoryle ($PO_3^{2-}$) ou un biotinyle ;
$R^2$ est OH, $NH_2$, H ou O(alkyle en $C_{1-4}$) ;
Xaa est un résidu d'acide aminé naturel ou non naturel possédant une chaîne latérale hydrophobe ;
Yaa est choisi parmi Gly, Lys, Lys-Gly, Lys*, Lys*-Gly, Zaa, Zaa*, Zaa-Gly et Zaa*-Gly, où Zaa est un acide aminé non naturel possédant une chaîne latérale amine, azide ou alcyne libre, et Zaa* est ledit acide aminé non naturel fonctionnalisé avec ou portant un fluorophore ; et
dans lequel Lys*, s'il est présent, est un résidu de lysine portant un fluorophore.

**2.** Composé ou sel acceptable pharmaceutiquement selon la revendication 1, dans lequel Xaa est choisi parmi Phe, phe, Ala, ala, Pro, pro, Gly, Val, val, Leu, leu, Ile, ile, Met, met, Tyr, tyr, Trp et trp ; dans lequel, éventuellement, Xaa est choisi parmi phe, ala, pro et Pro.

**3.** Composé ou sel acceptable pharmaceutiquement selon la revendication 1 ou la revendication 2, dans lequel Yaa est Gly.

**4.** Composé ou sel acceptable pharmaceutiquement selon la revendication 1 ou la revendication 2, dans lequel Yaa est Lys* ou Lys*-Gly.

**5.** Composé ou sel acceptable pharmaceutiquement selon la revendication 4, dans lequel le composé ou le sel est de Formule I.

**6.** Composé ou sel acceptable pharmaceutiquement selon la revendication 4 ou la revendication 5, dans lequel le fluorophore est choisi parmi l'acide carboxyfluorescéine et la cyanine5.

**7.** Composé ou sel acceptable pharmaceutiquement selon la revendication 1, dans lequel le composé est choisi parmi MFIGAF001, MFIGAF005, MFIGAF007 et MFIGAF008 :

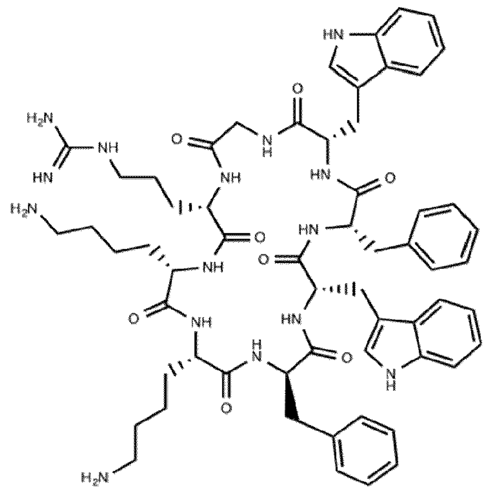

MFIGAF001

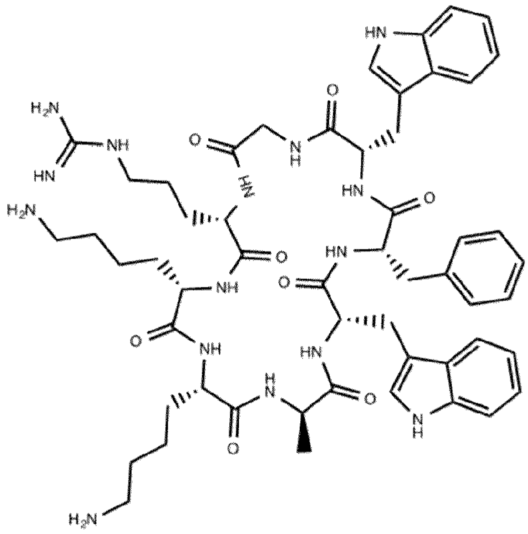

MFIGAF005

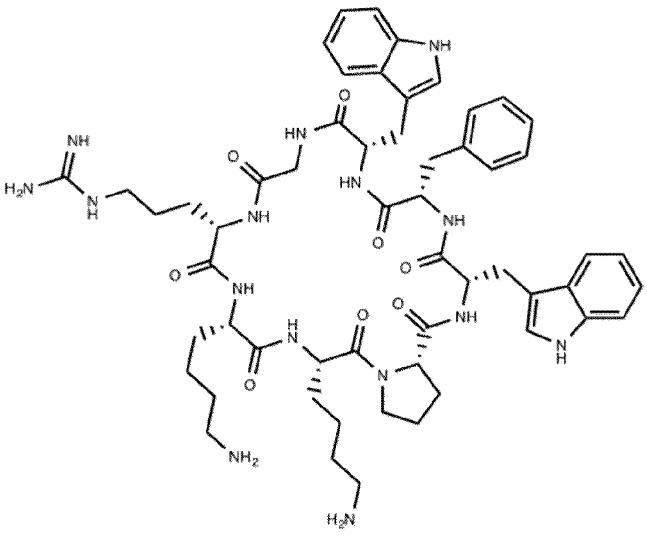

MFIGAF007

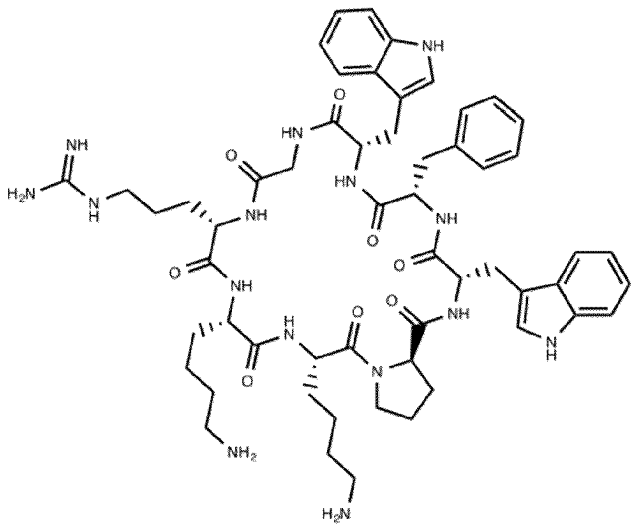

MFIGAF008.

8. Composé ou sel acceptable pharmaceutiquement selon la revendication 1, dans lequel le composé est le MFIGAF001 marqué par un fluorophore, dans lequel MFIGAF001 est :

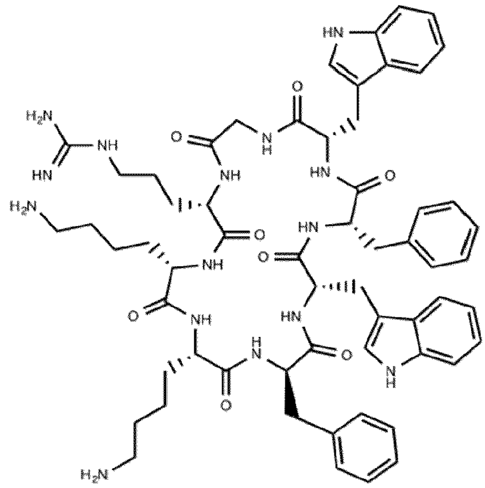

MFIGAF001.

9. Composé ou sel acceptable pharmaceutiquement selon la revendication 1, dans lequel le composé est choisi parmi MFIGAF000, MFIGAF002, MFIGAF003 et MFIGAF004

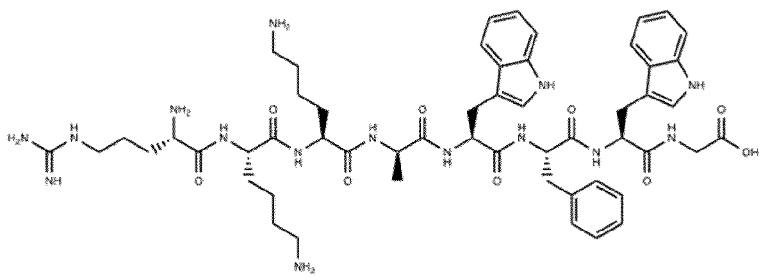

MFIGAF000

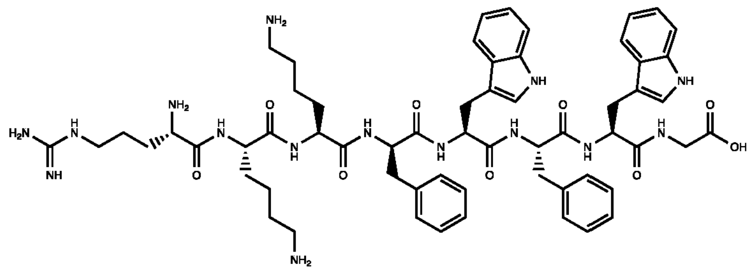

MFIGAF002

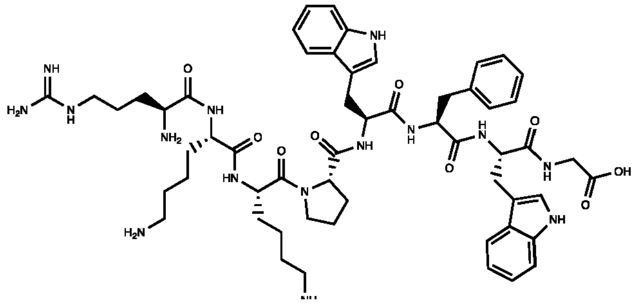

MFIGAF003

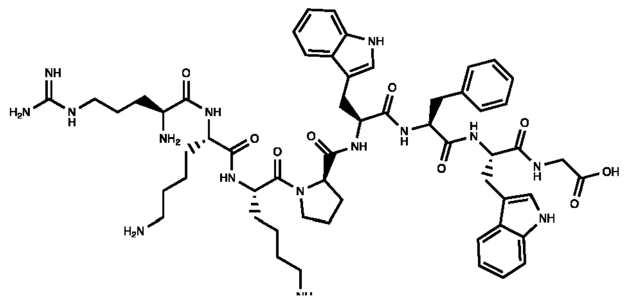

MFIGAF004.

**10.** Composition pharmaceutique comprenant le composé ou le sel de l'une quelconque des revendications 1 à 9 et un excipient acceptable pharmaceutiquement.

**11.** Composé ou sel selon l'une quelconque des revendications 1 à 9, ou composition pharmaceutique selon la revendication 10, destiné(e) à être utilisé(e) dans un procédé de traitement.

**12.** Composé ou sel selon l'une quelconque des revendications 1 à 9, ou composition pharmaceutique selon la revendication 10, destiné(e) à être utilisé(e) dans un procédé de traitement d'une infection fongique, d'une infection bactérienne ou d'une infection amibienne.

**13.** Composé ou sel destiné à être utilisé selon la revendication 12, dans lequel l'infection est une kératite fongique ou une kératite bactérienne.

**14.** Composé ou sel selon l'une quelconque des revendications 1 à 9, ou composition pharmaceutique de la revendication 10, ledit Yaa étant Lys*, Lys*-Gly, Zaa* ou Zaa*-Gly, destiné(e) à être utilisé(e) dans un procédé de diagnostic d'une infection fongique, bactérienne ou amibienne dans l'œil d'un patient, le procédé comprenant :

a) la mise en contact du composé, du sel ou de la composition avec l'œil du patient ;
b) l'inspection de l'œil du patient pour déterminer si la fluorescence est détectable ; et
c) **l'observation** de la fluorescence, si elle est présente, et le diagnostic d'une infection fongique, bactérienne ou amibienne chez le patient.

**15.** Procédé d'imagerie de la croissance microbienne dans un tissu à l'aide d'un composé ou d'un sel selon l'une quelconque des revendications 1 à 9, ou d'une composition pharmaceutique de la revendication 10, dans lequel Yaa est Lys*, Lys*-Gly, Zaa* ou Zaa*-Gly, le procédé comprenant :

a) la mise en contact du composé ou de la composition avec le tissu ;
b) la détection de la fluorescence du composé ou de la composition.

*Figure 1*

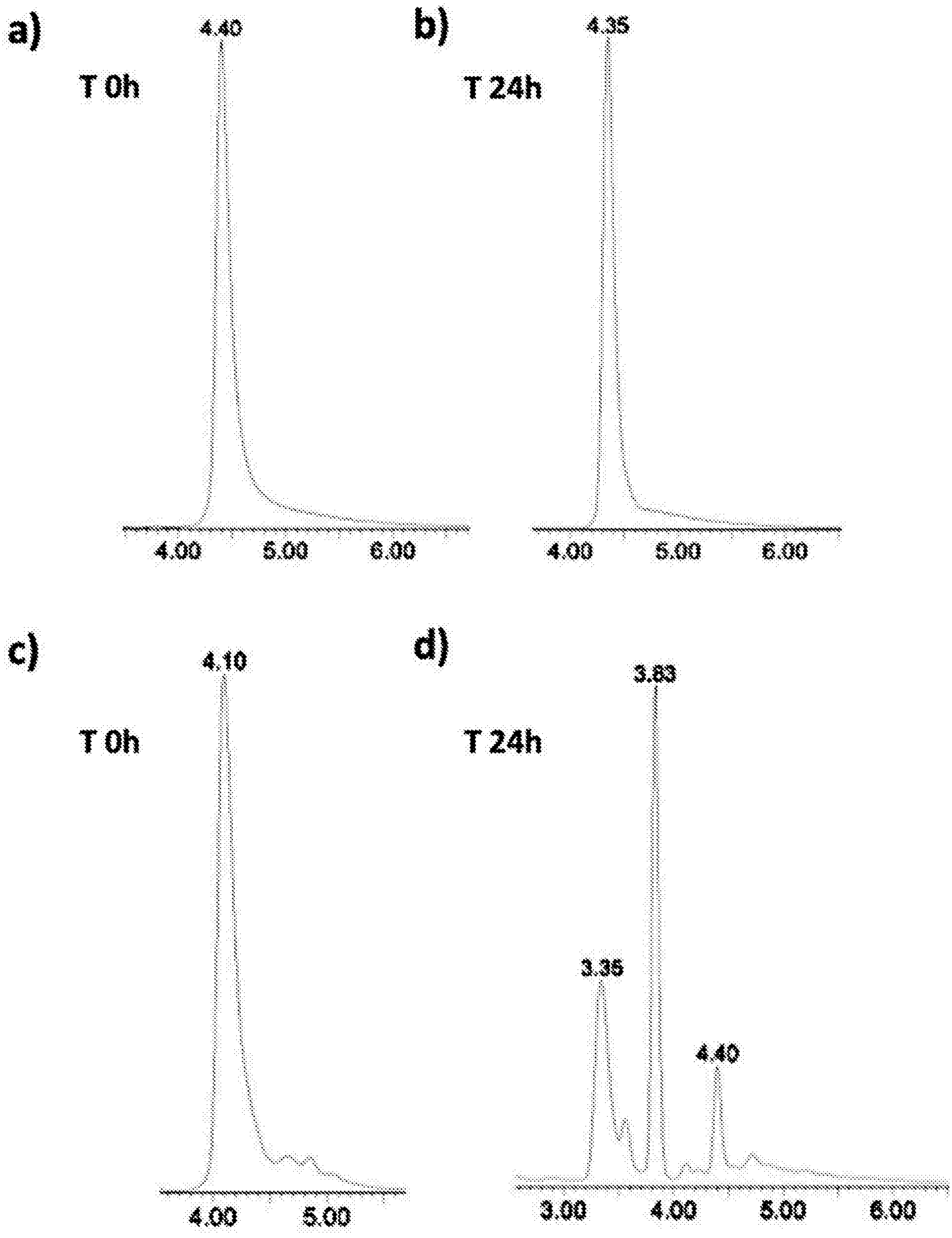
a)
T 0h
4.40
4.00
5.00
6.00
b)
T 24h
4.35
4.00
5.00
6.00
c)
T 0h
4.10
4.00
5.00
d)
T 24h
3.83
3.35
4.40
3.00
4.00
5.00
6.00

*Figure 2*

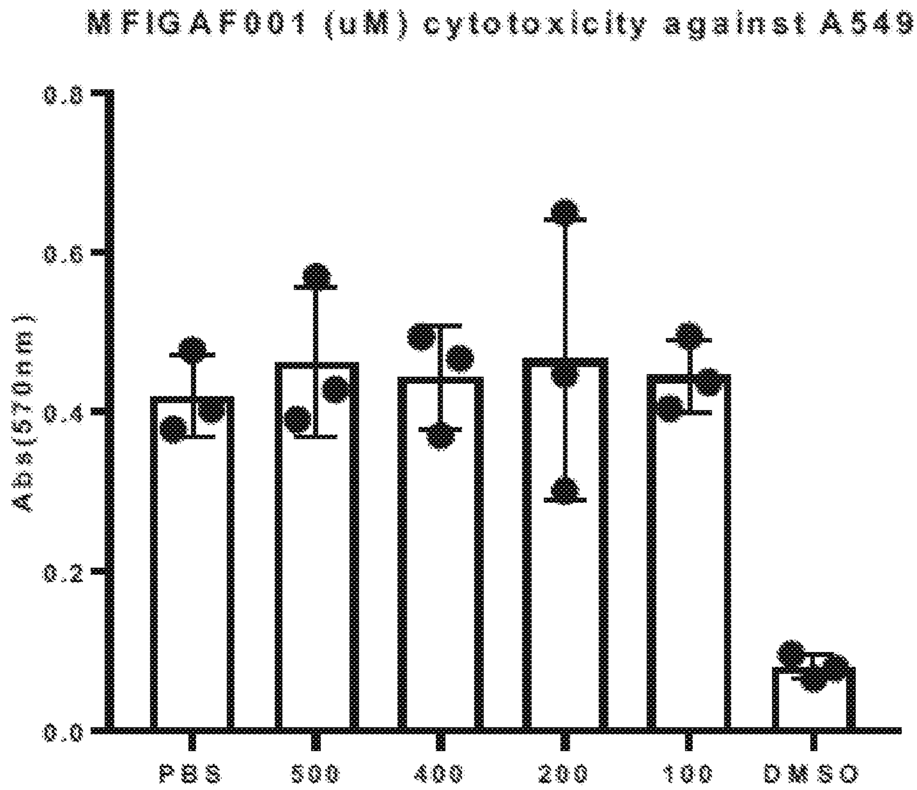
MFIGAF001 (uM) cytotoxicity against A549
Abs(570nm)
0.8
0.6
0.4
0.2
0.0
PBS
500
400
200
100
DMSO

*Figure 3*

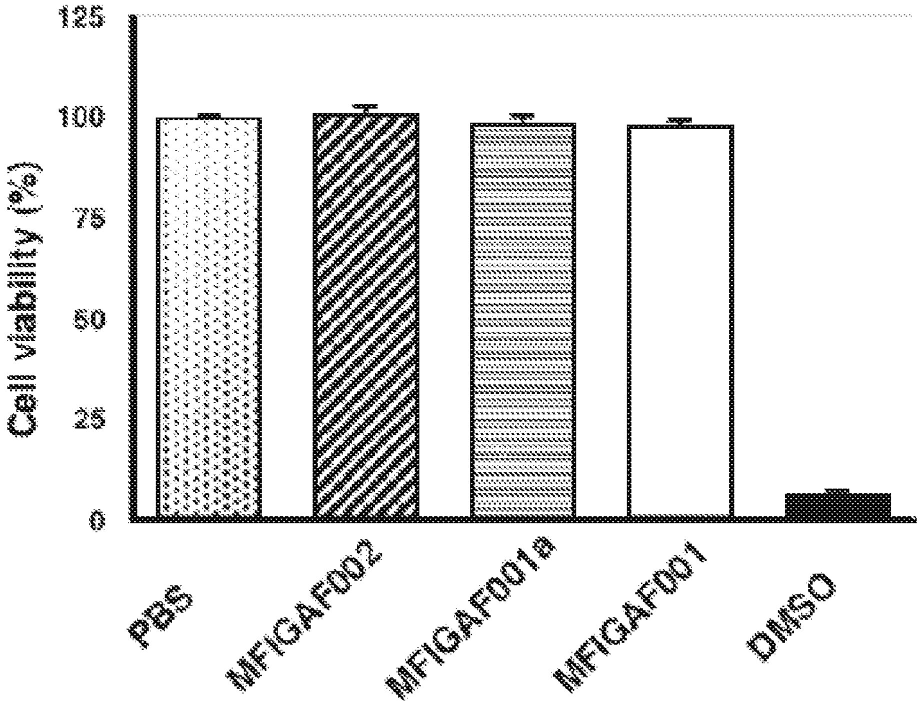
125
100
75
50
25
0
Cell viability (%)
PBS
MFIGAF002
MFIGAF001a
MFIGAF001
DMSO

*Figure 4*

a)

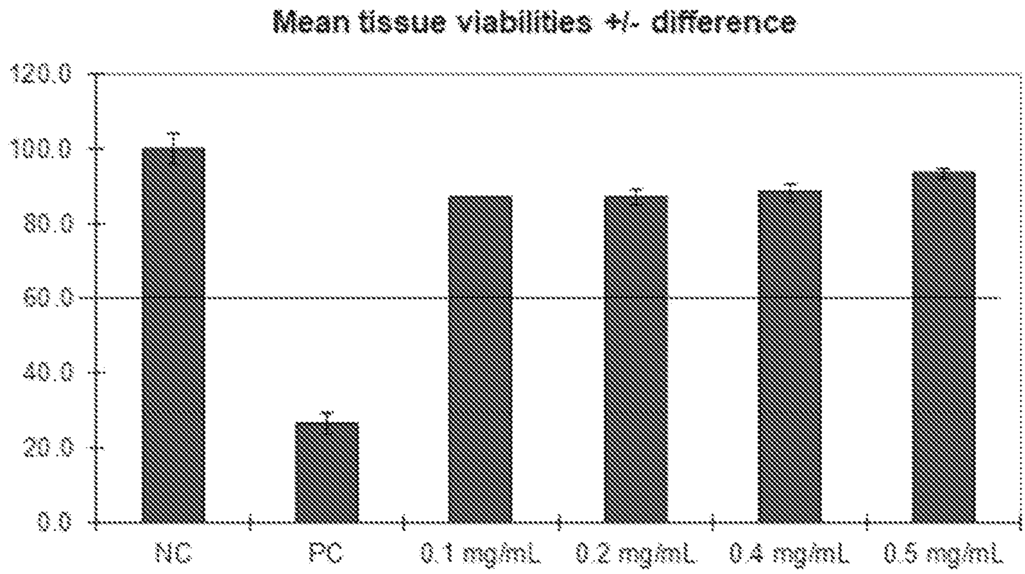
Mean tissue viabilities +/- difference
120.0
100.0
80.0
60.0
40.0
20.0
0.0
NC
PC
0.1 mg/mL
0.2 mg/mL
0.4 mg/mL
0.5 mg/mL

b)

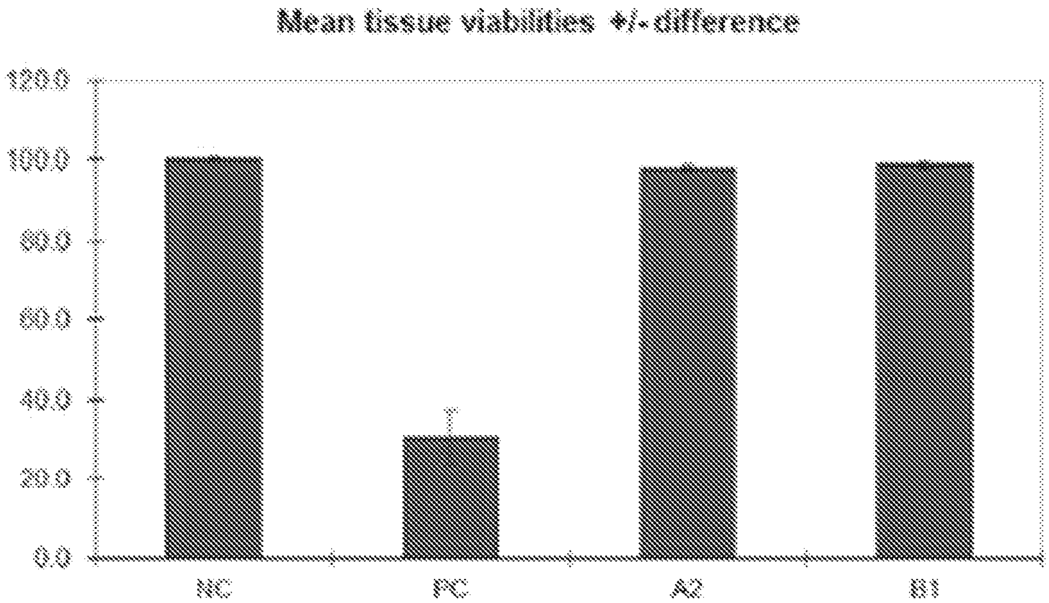
Mean tissue viabilities +/- difference
120.0
100.0
80.0
60.0
40.0
20.0
0.0
NC
PC
A2
B1

*Figure 5*

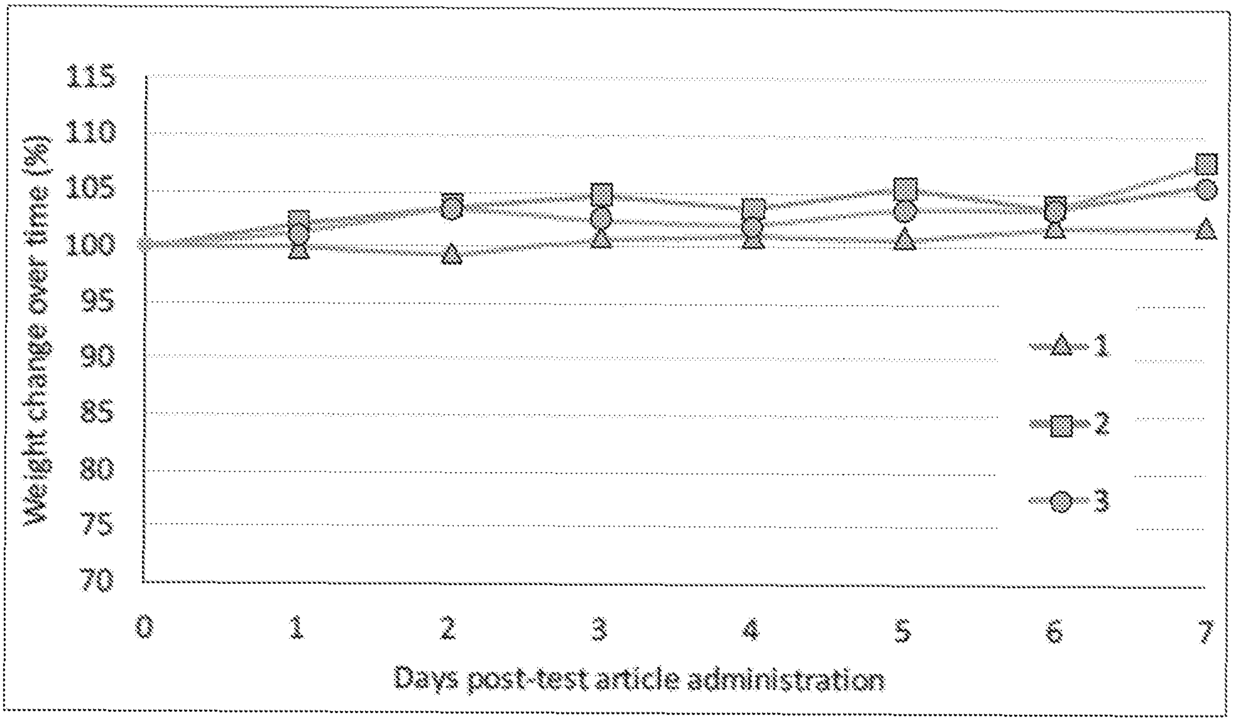
Weight change over time (%)
115
110
105
100
95
90
85
80
75
70
0
1
2
3
4
5
6
7
Days post-test article administration
1
2
3

## Figure 6

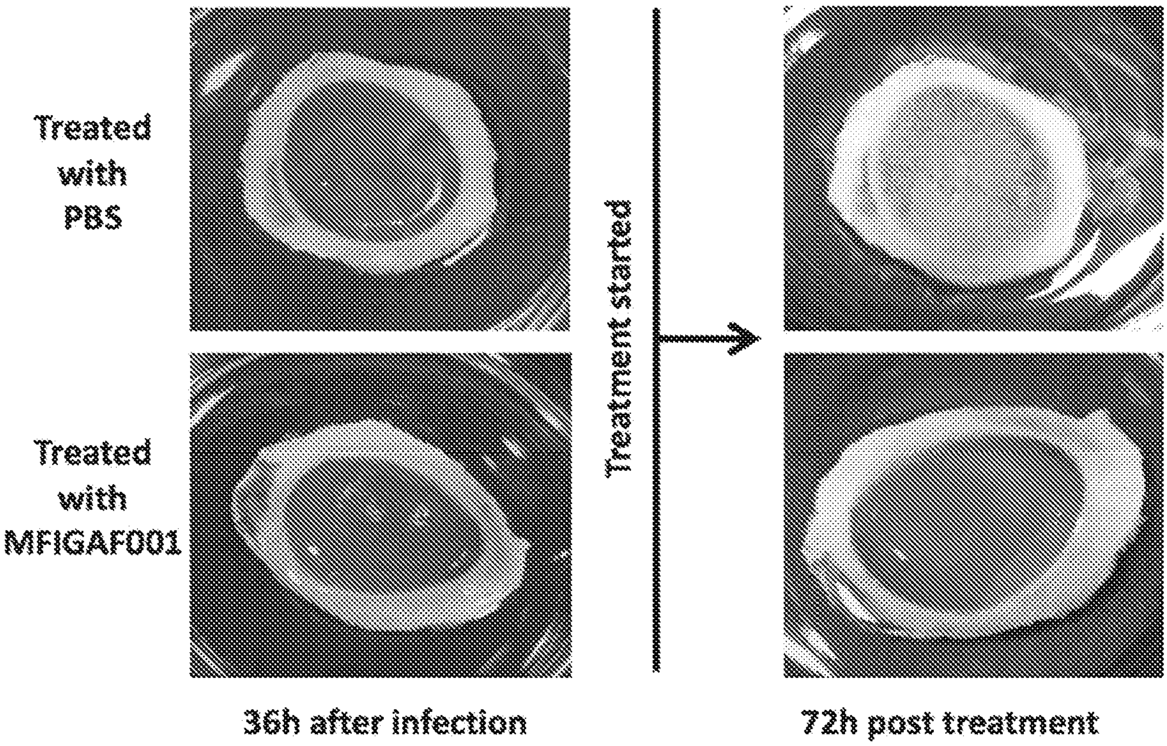
Treated
with
PBS
Treated
with
MFIGAF001
Treatment started
36h after infection
72h post treatment

*Figure 7*

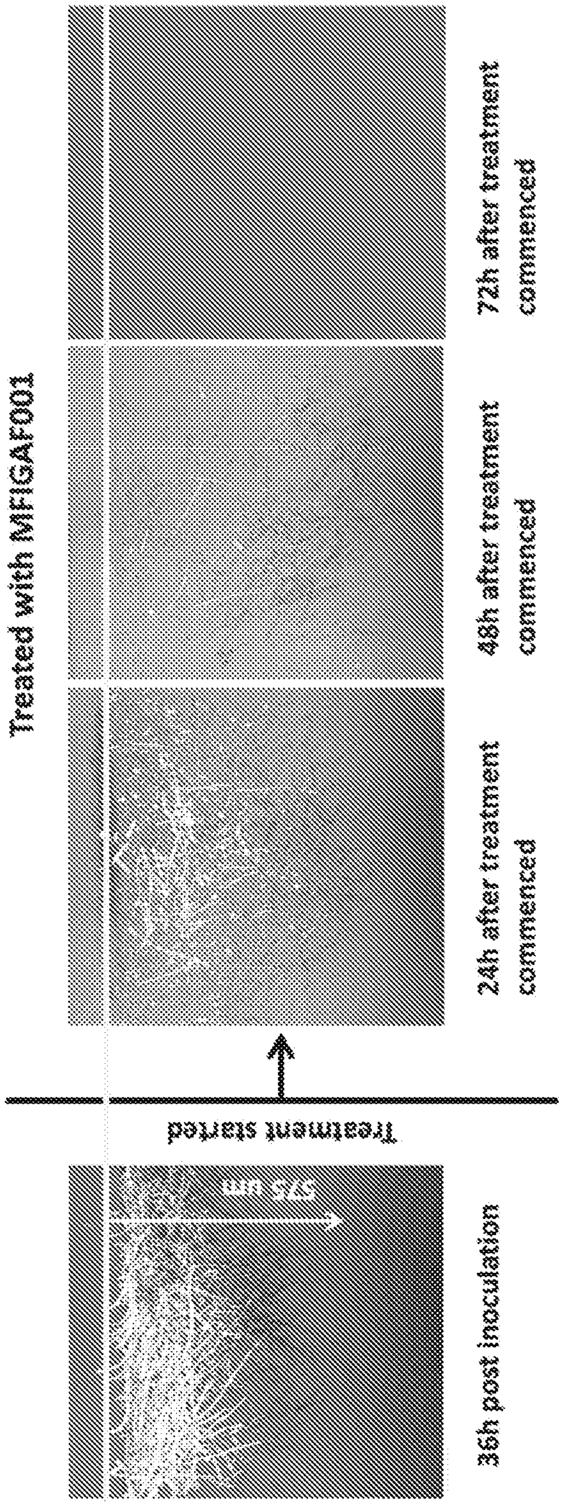
Treated with MFIGAF001
575 um
Treatment started
36h post inoculation
24h after treatment commenced
48h after treatment commenced
72h after treatment commenced

*Figure 8*

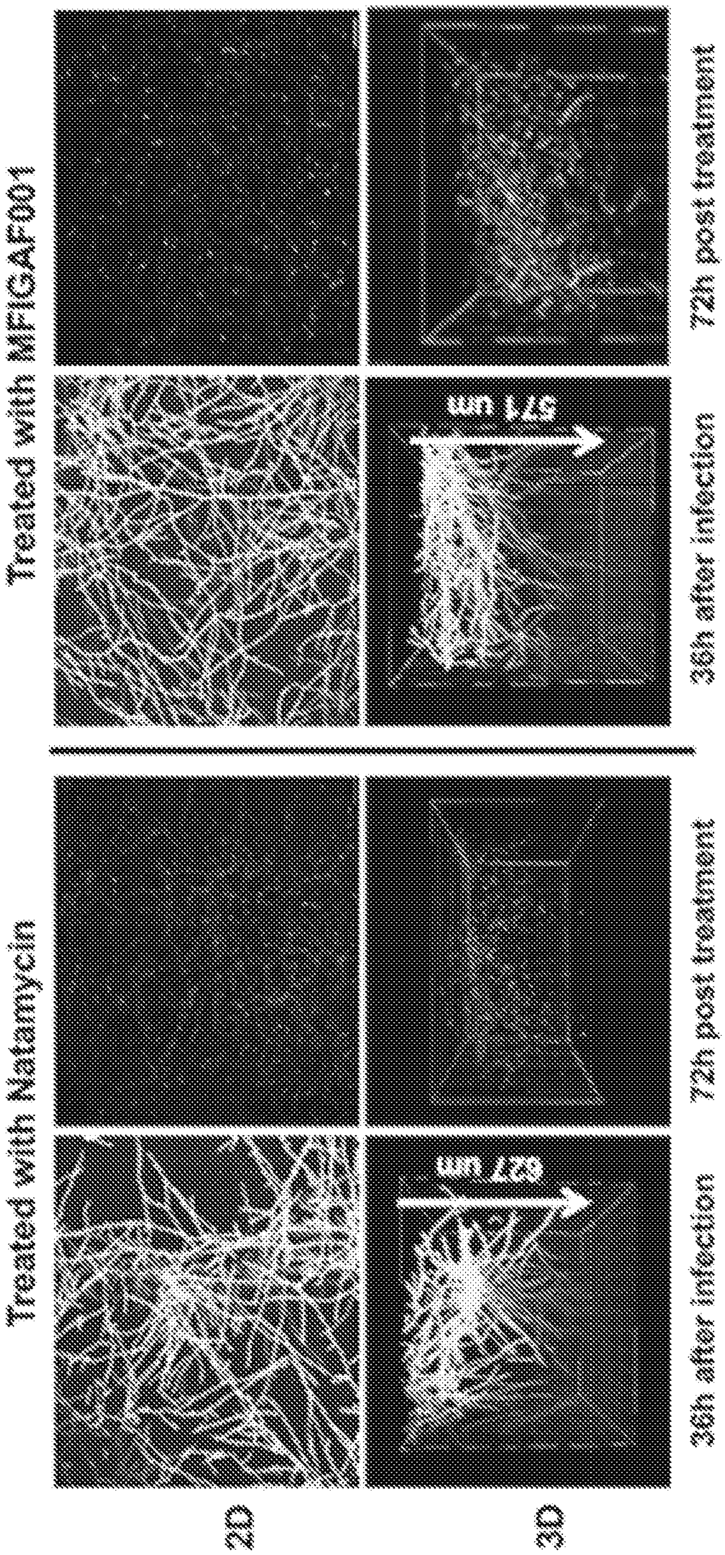
Treated with Natamycin
Treated with MFIGAF001
2D
3D
36h after infection
72h post treatment
36h after infection
72h post treatment
627 um
571 um

*Figure 9*

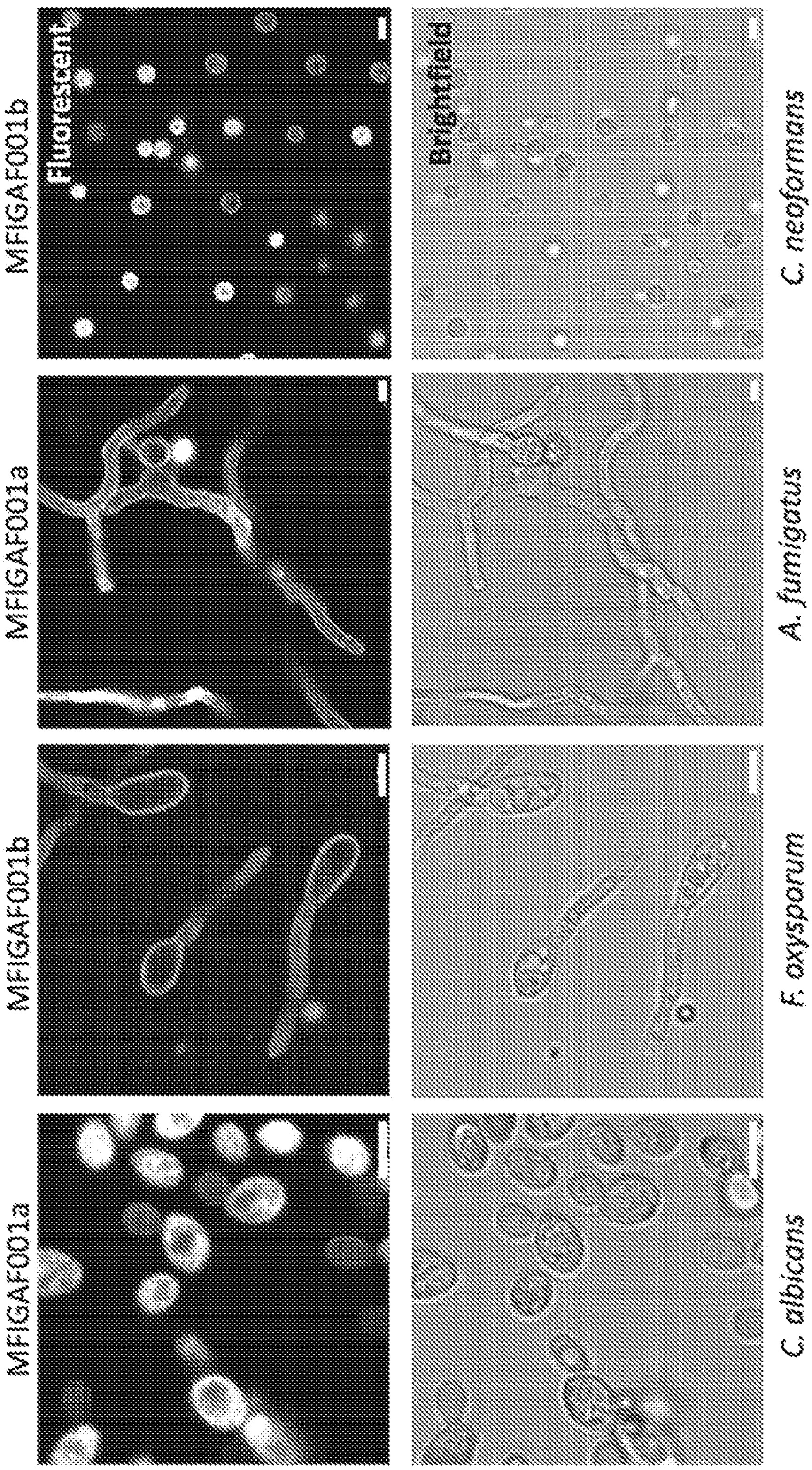
MFIGAF001b
MFIGAF001a
MFIGAF001b
MFIGAF001a
Fluorescent
Brightfield
*C. neoformans*
*A. fumigatus*
*F. oxysporum*
*C. albicans*

*Figure 10*

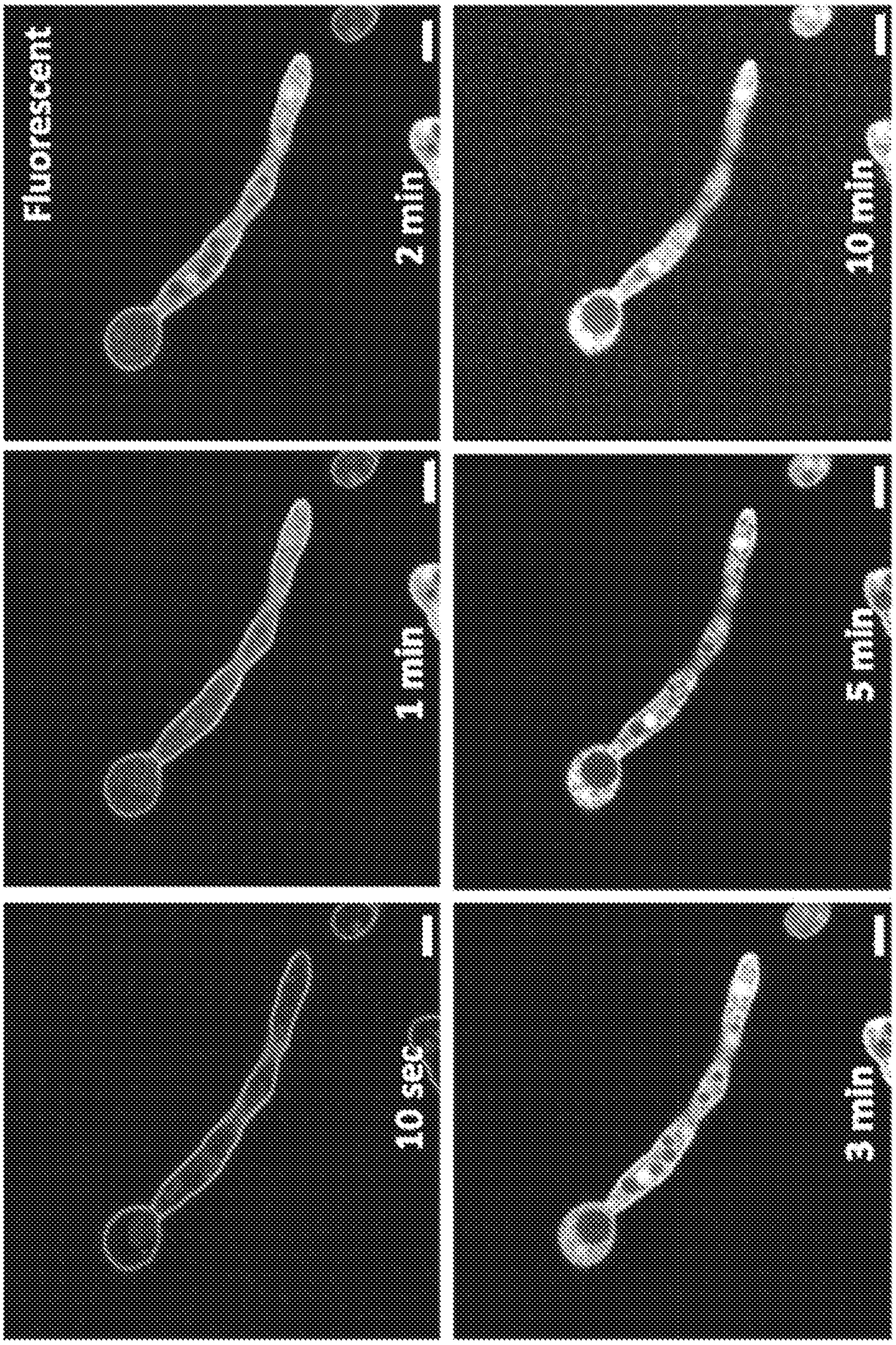

*Figure 11*

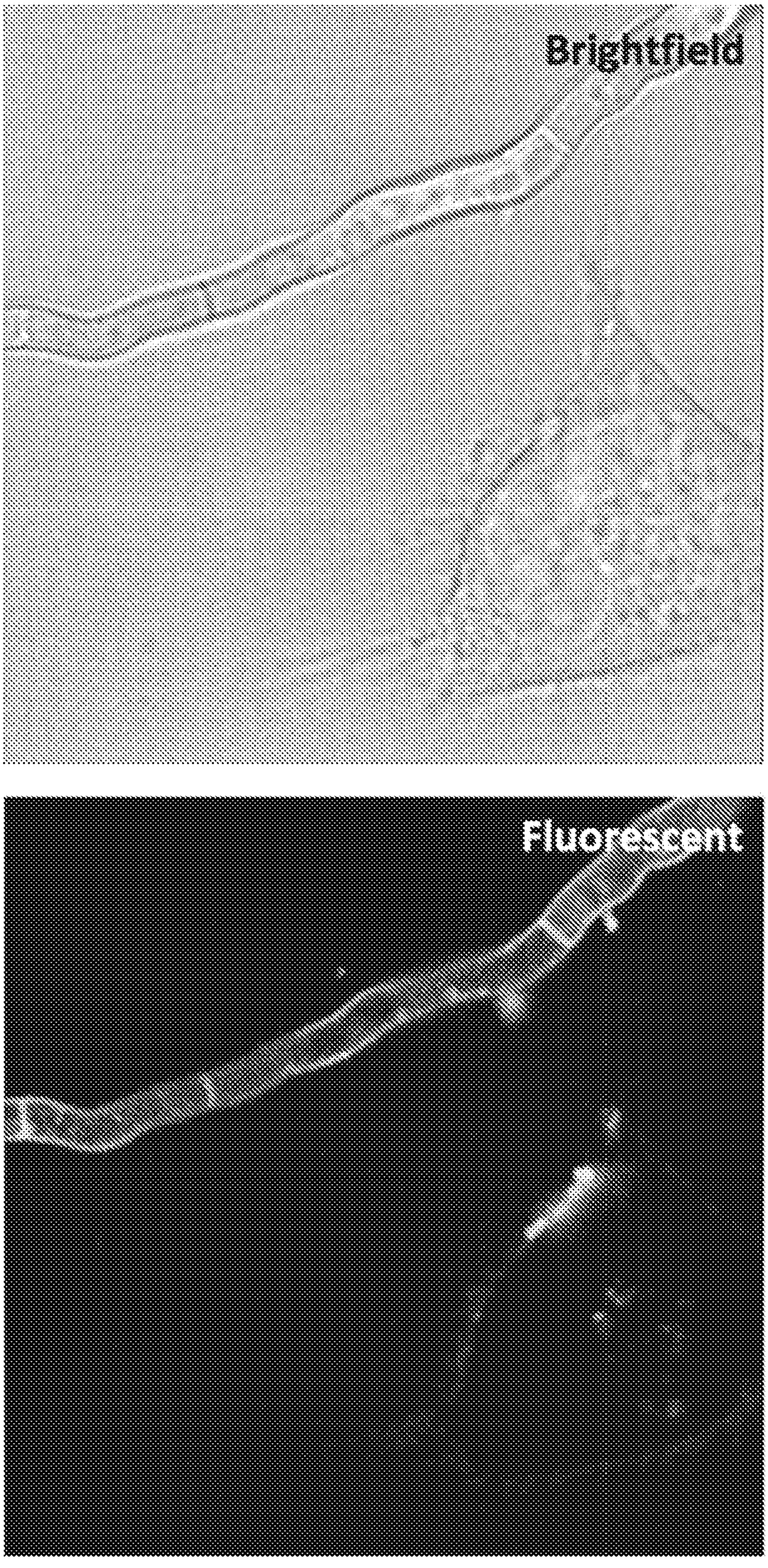

*Figure 12*

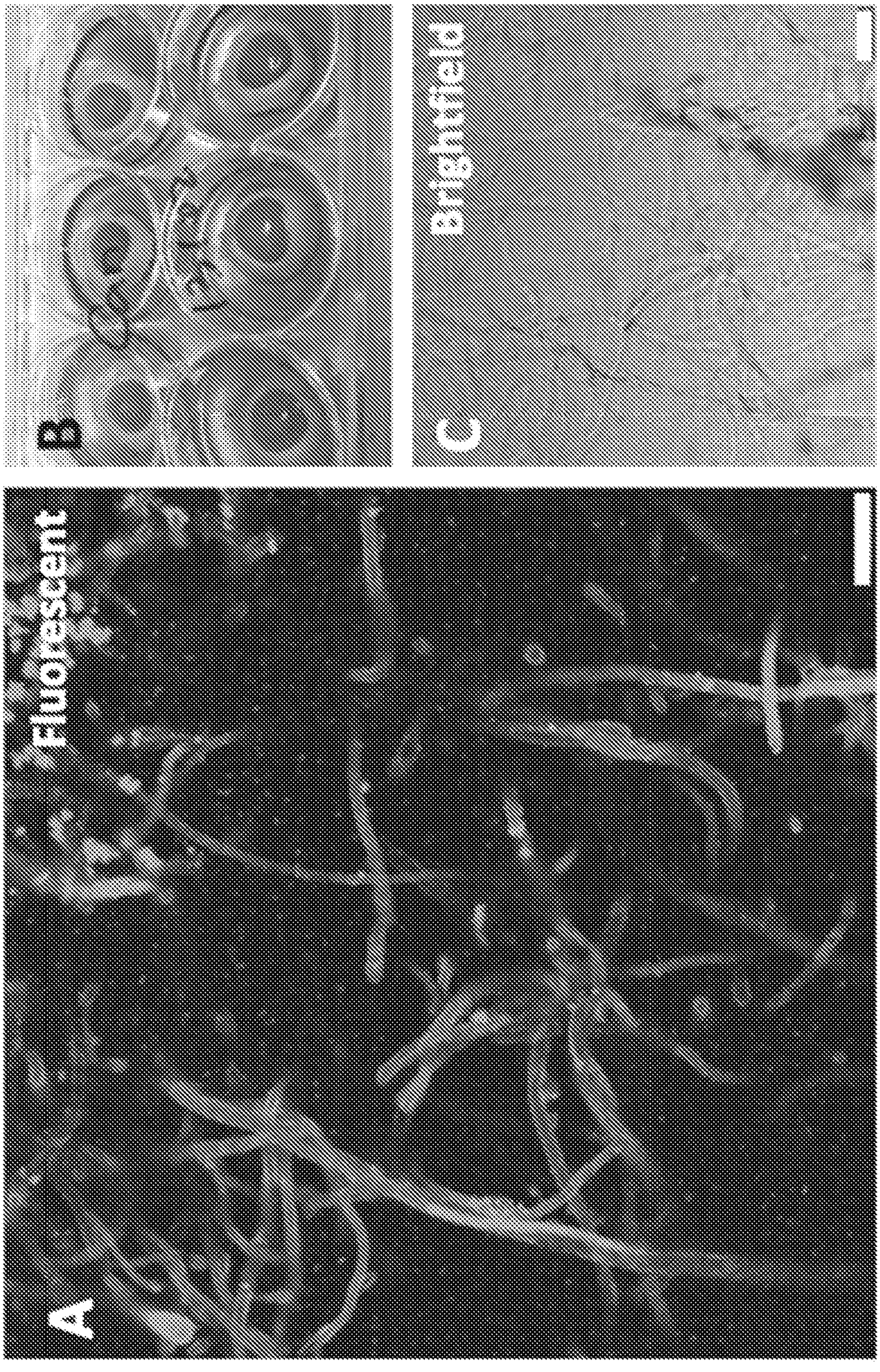

*Figure 13*

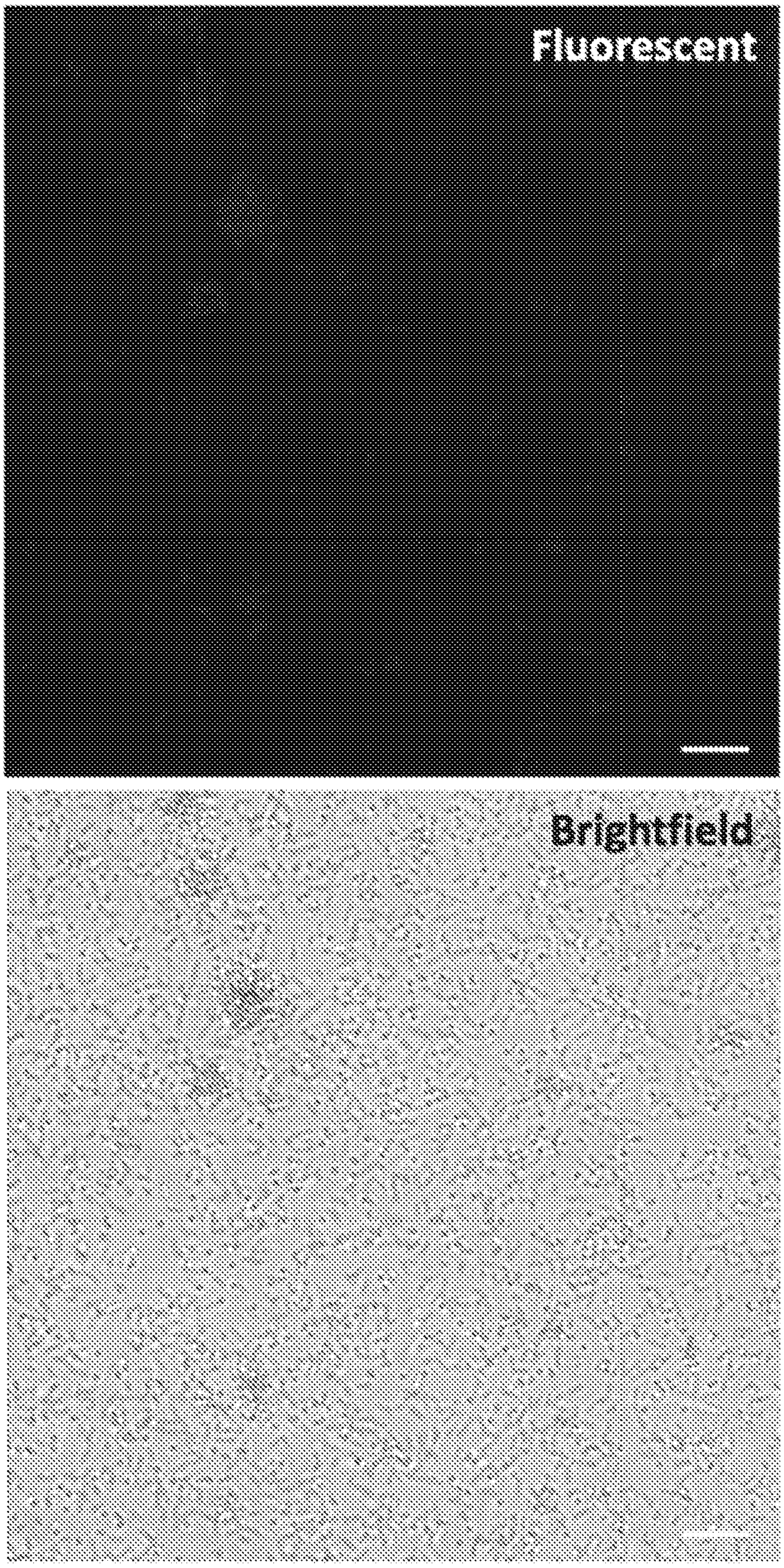

*Figure 14*

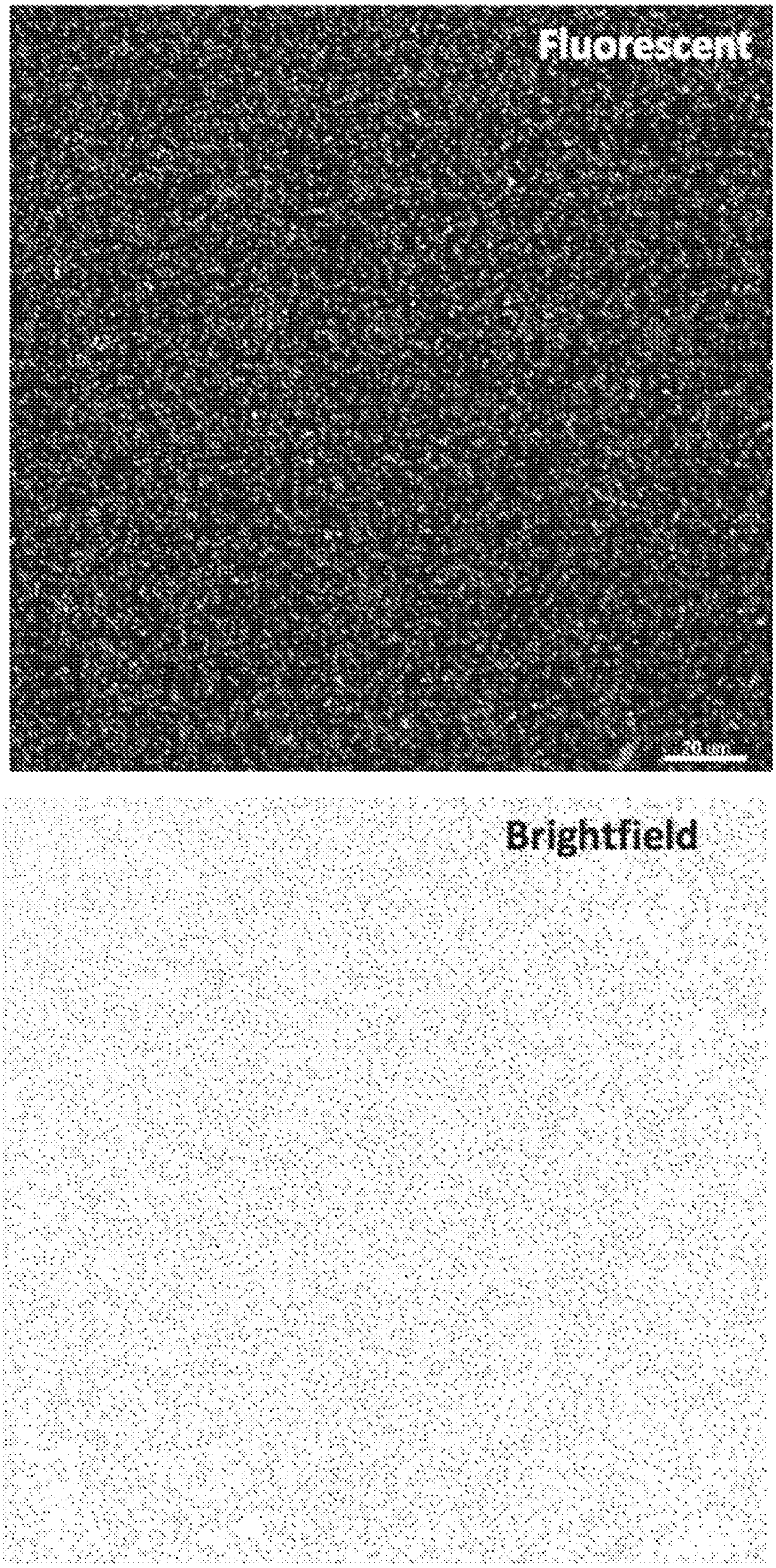

*Figure 15*

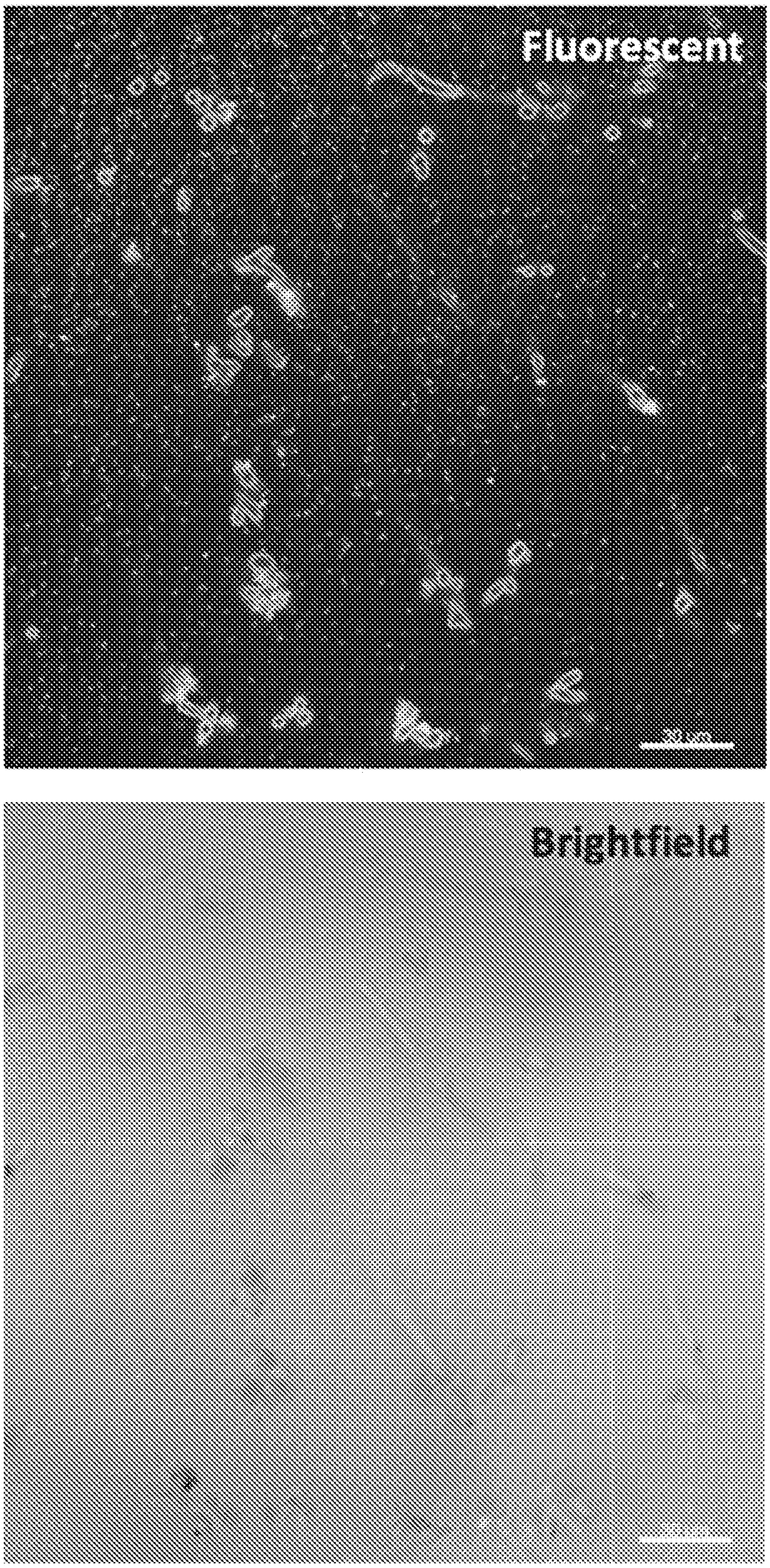

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2846840 A2
- WO 2013169932 A2
- ES 2191516 A1


### Non-patent literature cited in the description


- **LEE**. *Med Mycol.*, October 2018, vol. 56 (7), 796-802
- **MUÑOZ et al.** *Mol Microbiol.*, 31 May 2012, vol. 85 (1), 89-106
- **MENDIVE-TAPIA et al.** *Nature Protocols*, 2017, vol. 12, 1588-1619
- **ARIKAN et al.** In vitro susceptibility testing methods for caspofungin against Aspergillus and Fusarium isolates. *Antimicrob. Agents Chemother*, 2001, vol. 45, 327-330
- **BACON et al.** Acanthamoeba keratitis. The value of early diagnosis.. *Ophth*, 1993, vol. 100 (8), 1238-43
- **BROWN et al.** The Global Incidence and Diagnosis of Fungal Keratitis. *Lancet Infect. Dis.*, 2021, vol. 21 (3), e49-e57
- **BURTON et al.** Microbial keratitis in East Africa: why are the outcomes so poor?. *Ophthalmic Epidemiol.*, 2011, vol. 18 (4), 158-63
- **CHIDAMBARAM et al.** Prospective Study of the Diagnostic Accuracy of the In Vivo Laser Scanning Confocal Microscope for Severe Microbial Keratitis. *Ophthalmology*, 2016, vol. 123 (11), 2285-93
- **CHIDAMBARAM et al.** Epidemiology, risk factors, and clinical outcomes in severe microbial keratitis in South India. *Ophthalmic Epidemiol.*, 2018, 1-9
- **DALMON et al.** The clinical differentiation of bacterial and fungal keratitis: a photographic survey. *Invest. Ophthalmol. Vis. Sci.*, 2012, vol. 53 (4), 1787-91
- Molar absorptivity and values for proteins at selected wavelength of the ultraviolent and visible region. **FASMAN**. Practical Handbook of Biochemistry and Molecular Biology. 1989, 196-327
- **GROSDIDIER et al.** SwissDock, a protein-small molecule docking web service based on EADock DSS. *Nucl. Acids Res.*, 2011, vol. 39, W270-277
- **LEE et al.** *Exp. Eye Res.*, 2018, vol. 174, 51-58
- **MEHIO et al.** Identification of protein binding surfaces using surface triplet propensities. *Bioinformatics*, 2010, vol. 26, 2549-2555
- **NELSON et al.** Calcium measurement in living filamentous fungi expressing codon-optimized aequorin. *Mol. Microbiol.*, 2004, vol. 52, 1437-50
- **NEOH et al.** Clinical utility of caspofungin eye drops in fungal keratitis. *Int. J. Antimicrob. Agents*, 2014, vol. 44 (2), 96-104
- **OLDENBURG et al.** Evolution of practice patterns for the treatment of fungal keratitis. *JAMA Ophthalmol*, 2017, vol. 135 (12), 1448-1449
- **PERLIN et al.** *Med. Mycol.*, 2018, vol. 56 (7), 796-802
- **PETTERSEN et al.** UCSF Chimera--a visualization system for exploratory research and analysis. *J. Comput. Chem.*, 2004, vol. 25, 1605-12
- **PRAJNA et al.** The mycotic ulcer treatment trial: a randomized trial comparing natamycin vs voriconazole. *JAMA Ophthalmol.*, 2013, vol. 131 (4), 422-9
- **TUFT et al.** Microbial Keratitis. Royal College of Ophthalmologists, 2013
- **VOGEL**. A convenient growth medium for Neurospora (Medium N). *Microbial Genet. Bull.*, 1956, vol. 13, 42-43
- **SAMBROOK, J.** ; **RUSSEL, D.W**. Molecular Cloning, A Laboratory Manual. Cold Spring Harbor Laboratory Press